# EUROPEAN PATENT SPECIFICATION

(11) **EP 3 962 495 B1**
(45) Date of publication and mention of the grant of the patent: **31.07.2024**
(21) Application number: 20742923.4
(22) Date of filing: 09.06.2020
(51) Int. Cl.: A61K 31/728, A61K 31/738, C08B 37/08, C08J 3/24, C08L 5/08

(54) **NANOSTRUCTURED LINKED HYALURONIC ACID, METHOD FOR THE PRODUCTION THEREOF AND USE THEREOF AS EYE DROPS**
NANOSTRUKTURIERTE VERBUNDENE HYALURONSÄURE, VERFAHREN ZU IHRER HERSTELLUNG UND IHRE VERWENDUNG ALS AUGENTROPFEN
ACIDE HYALURONIQUE LIÉ NANOSTRUCTURÉ, PROCÉDÉ DE PRODUCTION DE CELUI-CI ET UTILISATION DE CELUI-CI EN TANT QUE GOUTTES OPHTALMIQUES

(30) Priority: 10.06.2019 IT 201900008505; 08.10.2019 IT 201900018266
(43) Date of publication of application: 09.03.2022
(73) Proprietor: Offhealth S.p.A., 50144 Firenze (FI) (IT)
(72) Inventor: ZANINI, Alessandro, I - 56017 San Giuliano Terme (PI) (IT); VELLANTE, Marco, I - 62029 Tolentino (MC) (IT); SODO, Eugenio, I - 00125 Rome (RM) (IT); CAVALLO, Giovanni, 00122 Roma Ostia (RM) (IT); FOSCHINI, Fulvio, I - 00124 Rome (RM) (IT)
(74) Representative: Sarpi, Maurizio
(86) International application number: PCT/IB2020/055408
(87) International publication number: WO 2020/250128

(56) References cited:
- KR-A- 20160 060 227
- US-A1- 2016 213 708

## Description

### Summary of the invention

The present invention relates to a method for the production of a cross-linked hyaluronic acid with BDDE (1,4-butanediol diglycidyl ether), to be used in the ophthalmic field, which, unlike what is known, is filterable, and therefore sterilizable, at 0.2 microns.

The research activity underlying the invention has surprisingly led to the selection of nanostructured cross-linked hyaluronic acids having a size of less than 200 nm, otherwise indicated below for brevity as "nano-linked." This allows to sterilize said acid by filtration at 0.2 µ, avoiding subjecting, in the ophthalmic field, the cross-linked hyaluronic acid and above all other components forming part of formulations containing said acid to a possible degradation, intended precisely for ophthalmic use, in particular, but not limited to, the treatment of dry eye syndrome, which are currently necessarily sterilized at 121°C for 15 minutes at a pressure of 1 atm.

A first object of the invention is therefore a preparation method which allows to identify and develop a cross-linked hyaluronic acid with BDDE of the type indicated above, which involves the following steps:
1) performing the cross-linking reaction starting from concentrations of hyaluronic acid sodium salt in a range between 1% and 4% with respect to the total weight of the composition and 20% BDDE with respect to the weight of the hyaluronic acid, thus maintaining the weight ratio between hyaluronic acid and BDDE always 5:1;
   - conducting the cross-linking reaction, in a basic medium, such as soda and potash, (from 1 to 0.2 molar) at a temperature of 30°C for 6 hours, which is the time necessary to optimize the cross-linking of the hyaluronic acid due to the effect of the BDDE and minimize as far as possible the possible hydrolysis of the hyaluronic acid due to the effect of the alkalinity;
   - partially neutralizing (or partially acidifying) the gel obtained by adding HCl or another acid which is compatible with the ocular surface, to bring it to a pH between 5.5 and 6.5;
   - leaving the neutralized cross-linked hyaluronic acid gel to rest at room temperature (25°C) for 6 days, so that the cross-linking process is complete; and
   - then diluting the same partially neutralized hyaluronic acid gel to 0.4% by weight with distilled water.
      - -This results in a filterable cross-linked product the particle size of which is between 8-50 nm, as measured in Light Scattering in Volume Mode, with a structure which is nanometric, and therefore attributable to the class of nanostructured systems.
   - It should be noted that the starting hyaluronic acid (sodium salt) not cross-linked under the same dilution conditions (0.4% w/w) has a particle size between 0.5 and 2 nm, always measured in Volume Mode with Light Scattering.

Within these limits, the cross-linking procedure of the present invention leads to a concentration of BDDE lower than 2 ppm which guarantees the non-cytotoxicity of the product.
- Another object of the invention is the procedure for producing and selecting new nanostructures for the preparation of sterile eye drops, by filtration at 0.2 µ, at 0.4% and 0.2% w/w nanostructured linked hyaluronic acid, (otherwise indicated hereinafter for brevity also as "nano-linked") which contain suitable buffers (buffer system, borate, Tris-Tris HCl, phosphate, citrate), which demonstrate an excellent stabilizing capacity of the lacrimal film, both against hyaluronic acid as such, and against hyaluronic acid derivatized with EDC.

Finally, a further object of the invention is the use of such eye drops in the treatment of dry eye syndrome, where the efficacy of these nanostructured linked eye drops is greater than that provided by eye drops consisting of only hyaluronic acid or hyaluronic acid (Icross^{®}) derivatized or modified with the EDC molecule.

### Field of the invention

Hyaluronic acid (HA) is one of the fundamental components of the connective tissues of man and other mammals. It gives the skin its particular properties of resistance and shape retention: in fact, the deficiency thereof causes a weakening of the skin with the formation of wrinkles and imperfections. Its concentration in tissues tends to decrease with advancing age due to the natural presence of particular enzymes, hyaluronidases, which catalyze the degradation of the HA chains.

From a chemical point of view, HA is a polymer which is considered linear, belonging to the class of glycosaminoglycans. The polysaccharide chain is produced by the aggregation of thousands of equal disaccharide units, formed by residues of glucuronic acid (a glucose derivative) and N acetylglucosamine, joined together by β-1,4 and β-1,3 glycosidic bonds. The structural formula is as follows:

### hyaluronic acid/sodium hyaluronate

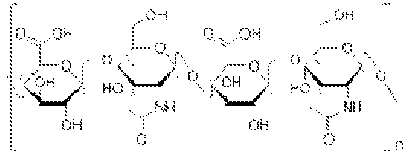

where the disaccharide unit repeats "n" times in the HA chain

HA chains can consist of 2,000-25,000 disaccharides which correspond to a relative molecular mass between 10⁶ - 10⁷ Da, and a length of 2 - 25 um.

All the carboxylic groups of HA are completely ionized in vivo, giving the molecule a high polarity, and therefore the ability to complex with many water molecules, reaching a high degree of hydration. In the amorphous matrix of a connective tissue the HA then takes care of maintaining the degree of hydration, turgidity, plasticity and viscosity of the unbranched polysaccharide chain produced by the condensation of thousands of disaccharide units formed in turn by residues of glucuronic acid and N-acetylglucosamine, linked together, alternately, by glycosidic bonds β1→4 and β1→3, as well as intramolecular hydrogen bonds, which stabilize the shapes.

### Applications of hyaluronic acid

The cosmetic and above all medical use of HA is linked to its chemical characteristic of complexing with many water molecules. In fact, thanks to this ability, when applied to skin or more generally to tissues, nasal mucosa, the surface of the eye, it is able to maintain the proper level of hydration even in the presence of a very low external humidity rate. This wetting action affects the mechanical properties of keratin, which becomes more flexible and elastic.

### Stability/fragility of hyaluronic acid

- Linear HA applied to the skin or other surfaces (nasal, ocular) or injected (in the case of fillers), as well as that naturally present in the dermis, is quickly degraded by hyaluronidases.
- The possible degradation is certainly accentuated in inflammatory sites, for example in synovitis, in inflammatory phenomena which more generally concern the joints: for this purpose, high concentrations of linear hyaluronic acid are administered, 1%, 2%, in some cases with other components such as glycerin, trehalose.
- The possible degradation is certainly present also in the case of products based on hyaluronic acid used as a filler for filling wrinkles or, more generally, imperfections affecting the face and lips.
- The possible degradation is certainly also accentuated in the case of the ocular surface in uveitis or at least partially in dry eye syndrome, where an inflammatory phenomenon cannot be excluded in more severe situations.

### Strategies to make hyaluronic acid more stable/resistant

- To slow down the process of physiological degradation of aging or resulting from different inflammatory situations, the HA is chemically modified. In this regard, various derivatization methods of hyaluronic acid are reported in literature which involve the modification of the carboxylic groups of hyaluronic acid and the modification of the N-acetyl groups of hyaluronic acid, in order to make the polymer molecule more resistant to enzyme attack. (1)
   - Other hyaluronic acid modification methods are also reported which involve the use of different compounds (DVS, epoxides, BDDE, EDC, etc.), which react with the hydroxyl groups (in position C6) of N-acetyl glucosamine, bind different molecules of hyaluronic acid to each other thus creating a reticulum: in this case it is called cross-linked or reticulated HA. In fact, in this form the β-1,4 glycosidic bonds, the breakage of which is catalyzed by hyaluronidases, are more protected, therefore the degradation process of the polysaccharide chain is slowed down if not blocked.

(2) The strategies to make hyaluronic acid more resistant which lead to a derivatized or cross-linked product must take into account that these products must be administered, in most cases, in a sterile form. This need, considering the viscosity and the structure of the products, can be solved only with fluent steam sterilization (121°C for at least 15 minutes at the pressure of 1 atm). However, the effect of the high temperature can destabilize the derivatized hyaluronic acids and in any case decrease the compactness of the cross-linked products. Furthermore, especially in products such as eye drops, the thermal sterilization degrades any excipients, such as proteins, vitamins, and amino acids.

### Background art

To summarize the above, it can be understood how sterilization by filtration at 0.2 microns is limited to very low linear hyaluronic acid concentrations, while in practice it is not pursuable with cross-linked hyaluronic acid solutions.

See for example document ΣCR 2016 0060227, which describes products comprising crosslinked BDDE hyaluronic acid, and US 2016/213708 A1 relating to a sterile eye product consisting of active ingredients, linear hyaluronic acid (not crosslinked, (polyvinipyrrolidone) (PVP).

The impossibility of sterilizing cross-linked hyaluronic acid by filtration at 0.2 microns stems from the fact that the cross-linking agents, reacting with some functional groups (the hydroxyls in position C6 and the carboxylic groups) of different chains of hyaluronic acid create a sufficiently rigid compact structure which prevents the filterability of the product, especially of a product conceived as a filler.

To better clarify what stated above, a structure of hyaluronic acid cross-linked with BDDE is reported below.

Structural formula 1 is shown below.

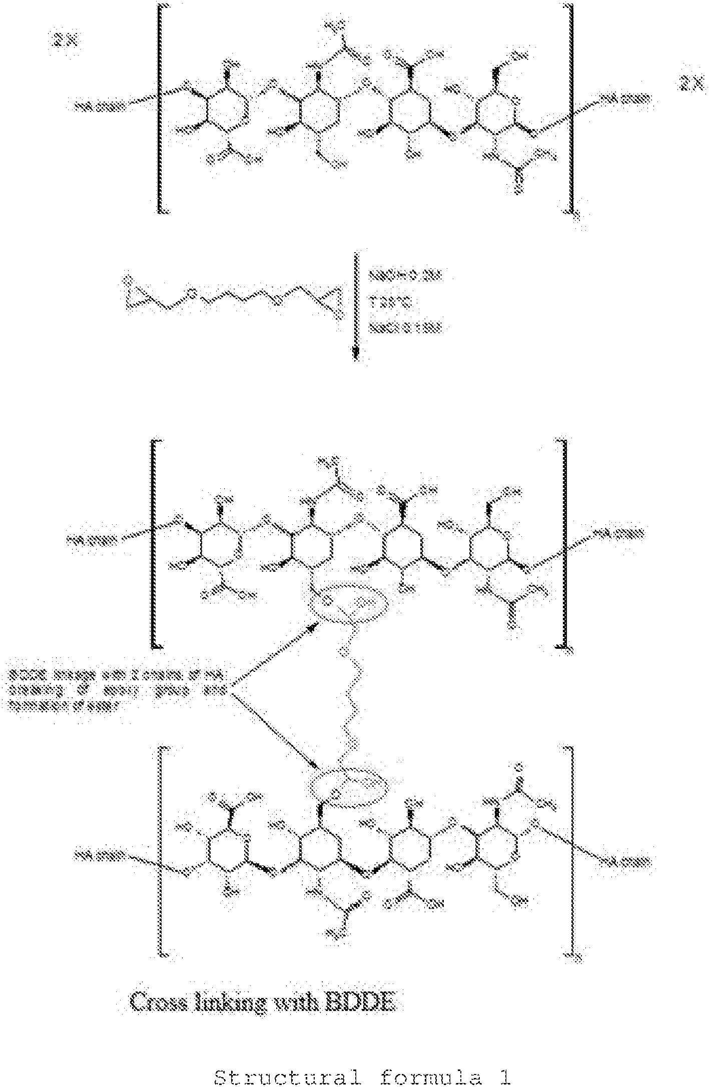

### Description of the strategy for producing a cross-linked hyaluronic acid with BDDE sterile by filtration at 0.2 microns

In consideration of what known in literature, the inventors of the present patent application started with the production of a hyaluronic acid gel cross-linked with BDDE (1).

One method frequently used today for cross-linking HA in hydrogels for products currently on the market (Schanté et al., 2011) is the reaction with 1,4-butanediol diglycidyl ether (otherwise BDDE), in an alkaline medium to obtain a stable covalent ether between HA and the cross-linker (Mälson & Lindqvist, 1986) .

In the reaction, nucleophilic groups of HA react with the epoxy groups of BDDE. In theory, six sites in each HA-disaccharide unit are available for the reaction with BDDE. Starting from that the deprotonated hydroxyls are much stronger nucleophiles than the anionic carboxyl group and the amide, the present inventors assumed that the hydroxyl groups are the most probable reaction sites, forming ether bonds with the cross-linker. Therefore, any ester formed with the carboxylate group of HA will probably be hydrolyzed under alkaline conditions. The basic idea was therefore to identify a process comprising the following steps;
1) Preparing a hyaluronic acid gel (sodium salt) in an alkaline medium cross-linked with BDDE;
2) Diluting said gel to a final concentration which would allow its filterability at 0.2 microns;
   - 3) Homogenizing the gel thus diluted using a cutting force produced by a homogenizer; and
   - 4) Sterilizing the homogenized product by filtration at 0.2 microns.

An experimental activity was therefore carried out aimed at defining the optimal values of the process parameters to obtain solutions of cross-linked hyaluronic acid which is sterilizable by filtration at 0.2 microns.

The description of the invention will be better followed by referring to the accompanying drawings, in which:

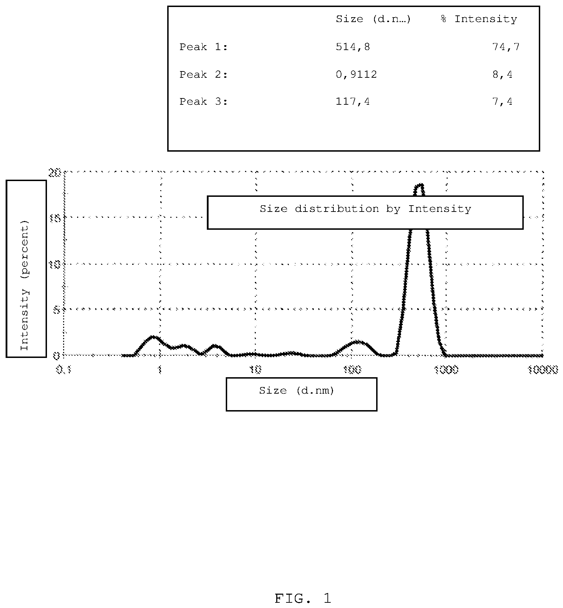

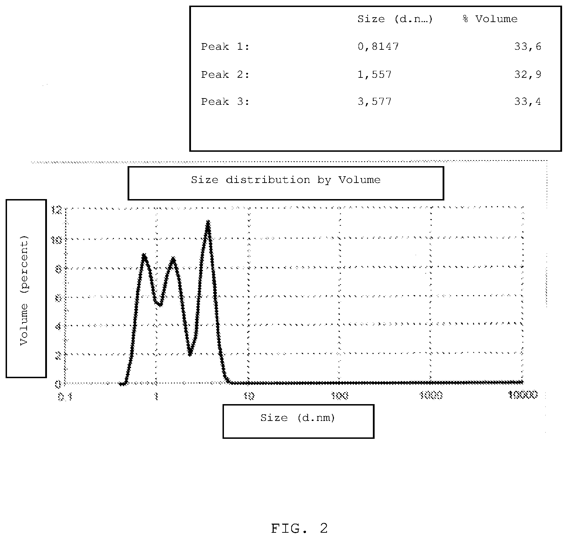

Figs. 1 and 2 are the graphs related to the distribution analysis of Intensity and Volume in Light scattering respectively of a sample of the starting hyaluronic acid produced at 0.4%, which is filterable per se and therefore sterilizable at 0.2 microns before being cross-linked;

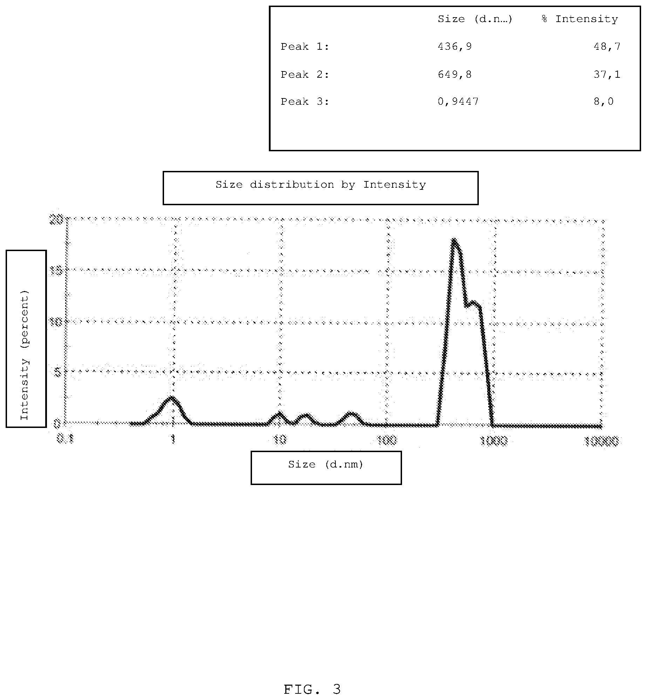

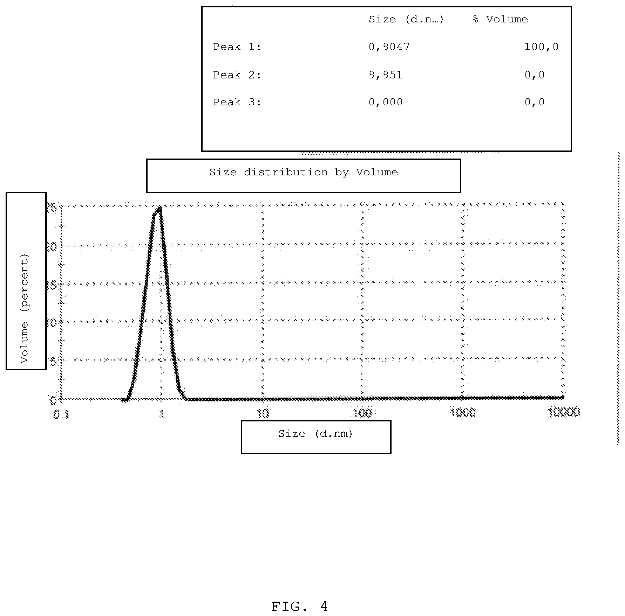

figs. 3 and 4 are the graphs related to the same distribution analysis of Intensity and Volume respectively of a sample of the starting hyaluronic acid produced at 0.2%, before being cross-linked;

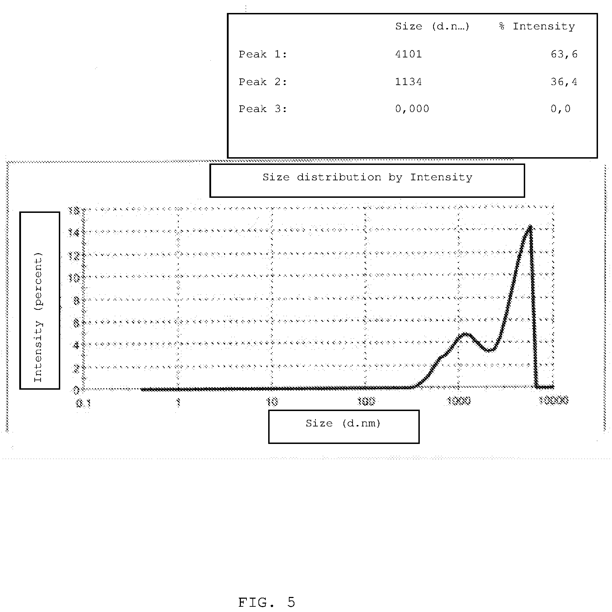

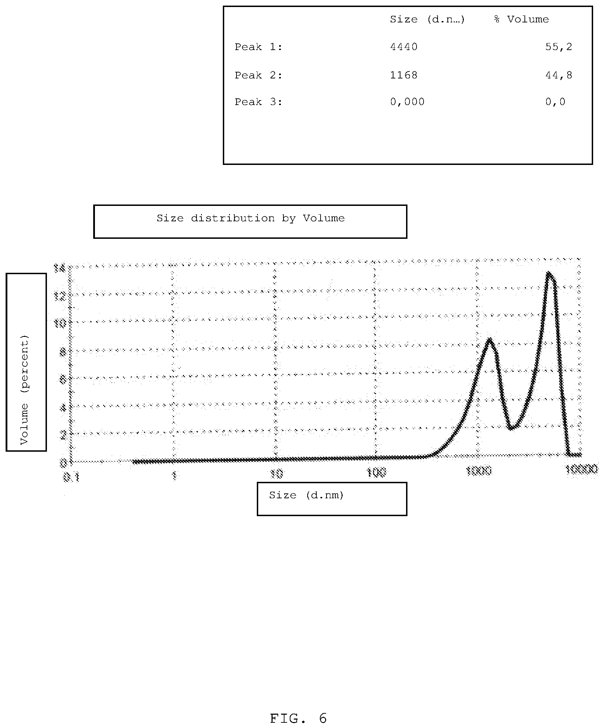

figs. 5 and 6 are the graphs related to the same distribution analysis of Intensity and Volume respectively of a sample of cross-linked hyaluronic acid at 10% with BDDE, diluted to a final concentration of 0.2% w/w after homogenization, which is not filterable at 0.2 microns;

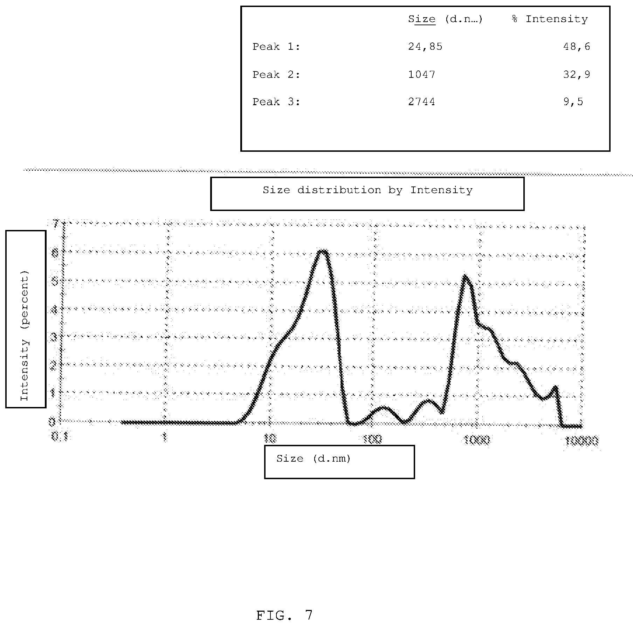

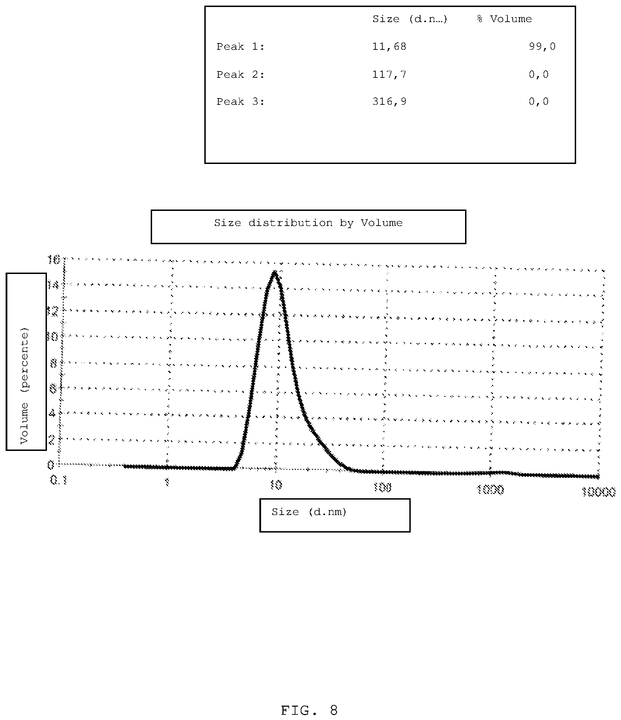

figs. 7 and 8 are the graphs related to the same distribution analysis of Intensity and Volume, respectively, of a sample of cross-linked hyaluronic acid at 4% with BDDE, diluted to a final concentration of 0.2% w/w after homogenization, which is instead filterable at 0.2 microns, and therefore sterilizable;

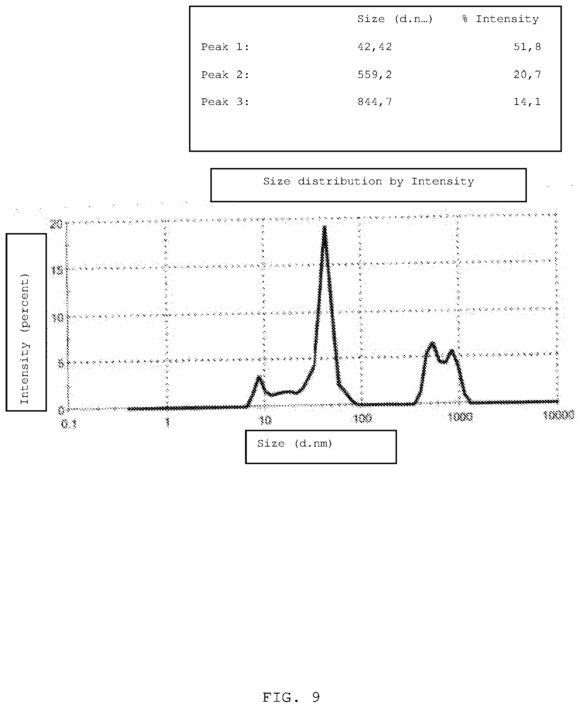

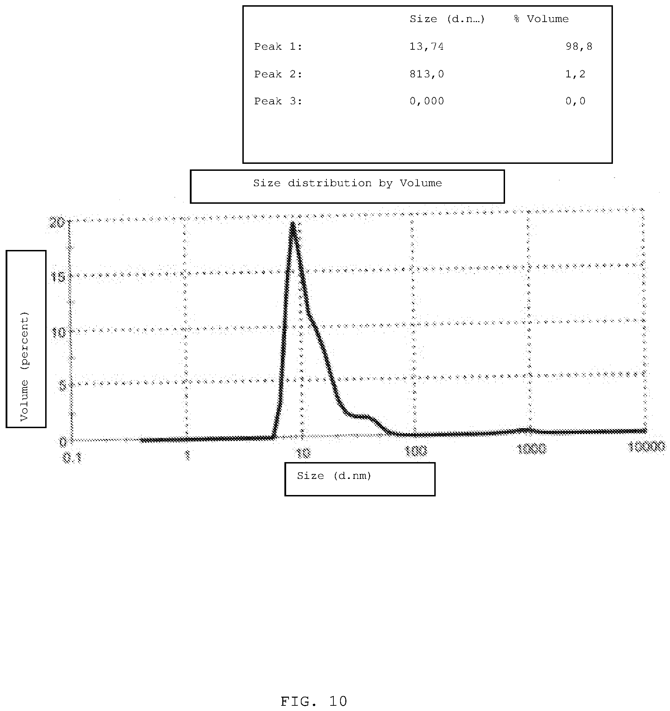

figs. 9 and 10 are the graphs related to the same analyses of a hyaluronic acid sample cross-linked with BDDE starting from a concentration of 1% hyaluronic acid in the cross-linking reaction, in 1 molar soda, where the ratio between BDDE and hyaluronic acid sodium salt is always 1/5 w/w, which is also filterable at 0.2 microns and therefore sterilizable;

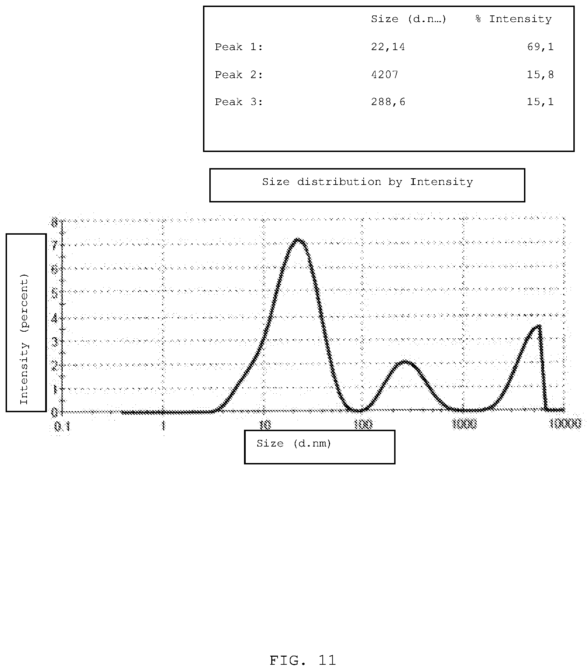

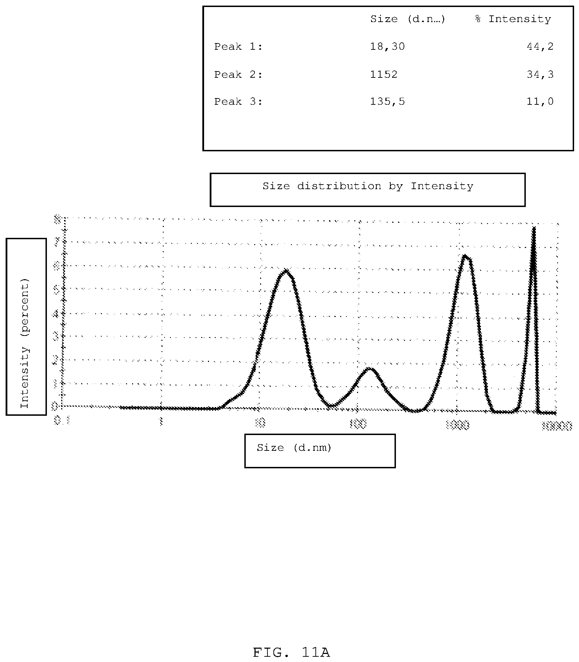

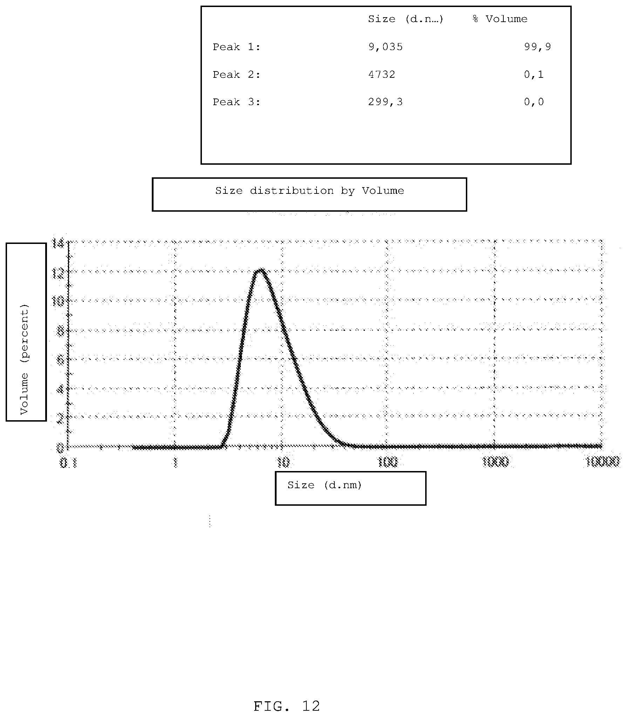

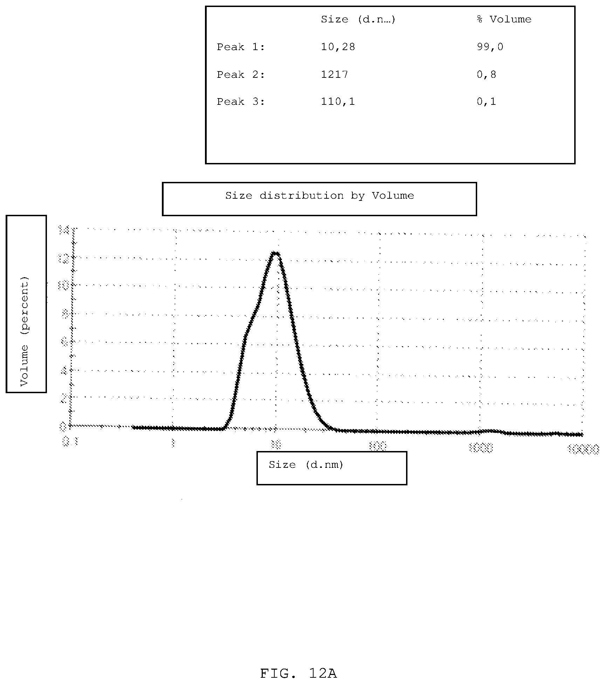

figs. 11 and 12 are the graphs related to the same distribution analysis of Intensity and Volume respectively of a sample of cross-linked hyaluronic acid at 4% with BDDE (i.e. starting from a cross-linking reaction with hyaluronic acid at 40), in 0.2 molar soda and diluted to 0.2%, where the ratio between BDDE and hyaluronic acid sodium salt is 1/5; this product is filterable at 0.2 microns and therefore sterilizable;
figs. 11a and 12a are the graphs related to the same distribution analysis of Intensity and Volume respectively of a sample like the preceding one, of cross-linked hyaluronic acid at 4% with BDDE (i.e. starting from a cross-linking reaction with hyaluronic acid at 40), in 0.2 molar soda and diluted to 0.2%, where the weight ratio between BDDE and hyaluronic acid sodium salt is 1/5; where this product has not been subjected to filtration at 0.2 microns;

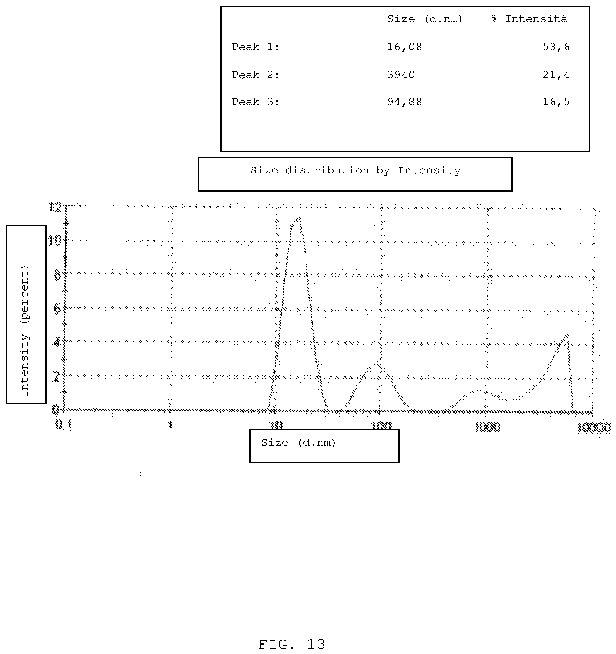

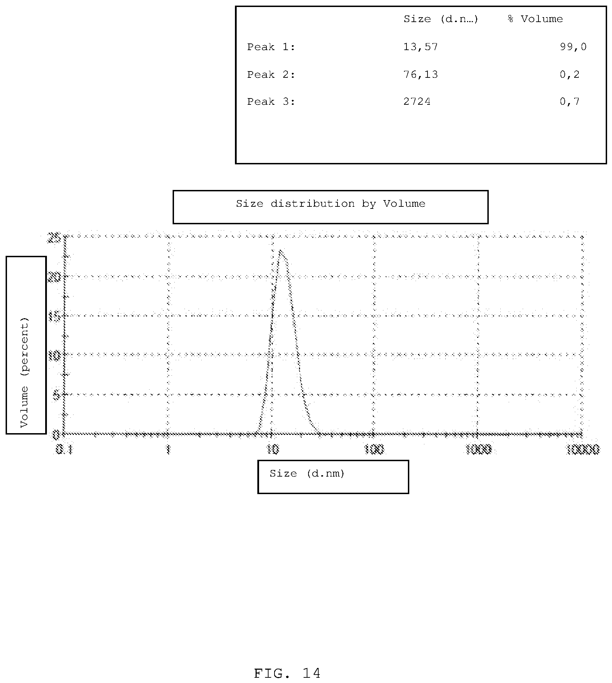

figs. 13 and 14 are the graphs related to the same distribution analysis of Intensity and Volume respectively of a sample of eye drops produced with cross-linked hyaluronic acid at 4% in 1 molar sodium, diluted to 0.4% in distilled water, in borate buffer;

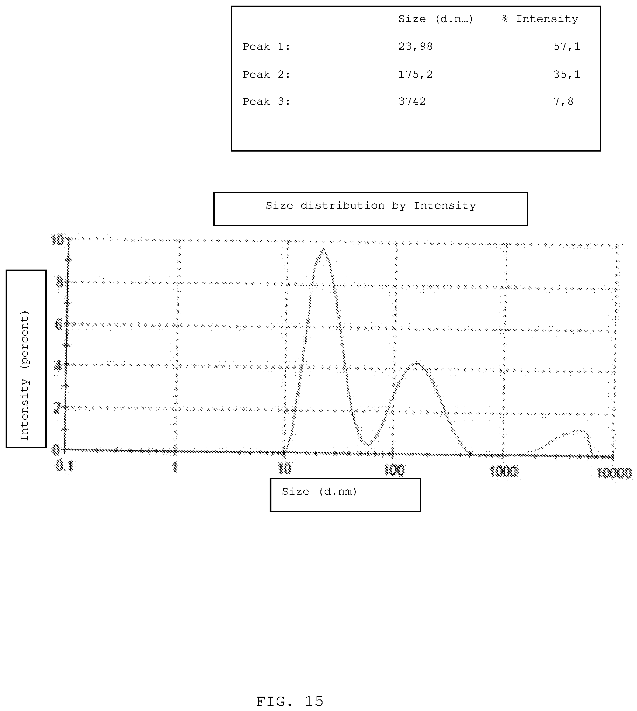

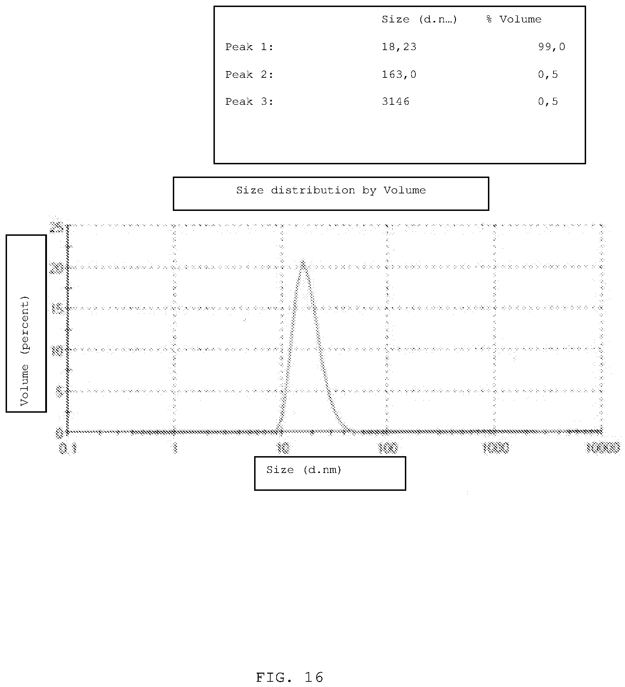

figs. 15 and 16 are the graphs related to the distribution analysis of Intensity and Volume respectively of a sample of eye drops produced with cross-linked hyaluronic acid at 4% in the cross-linking step in alkaline medium of 0.2 molar NaOH in borate buffer;

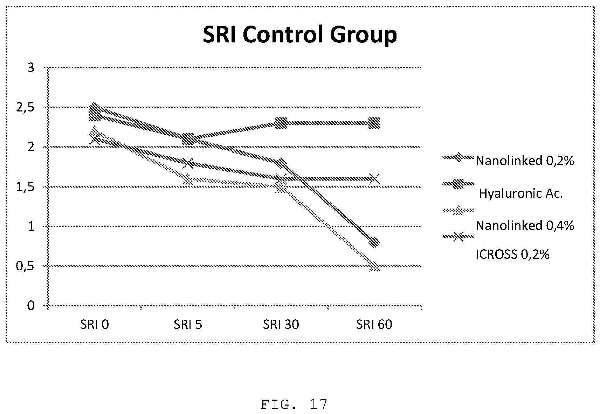

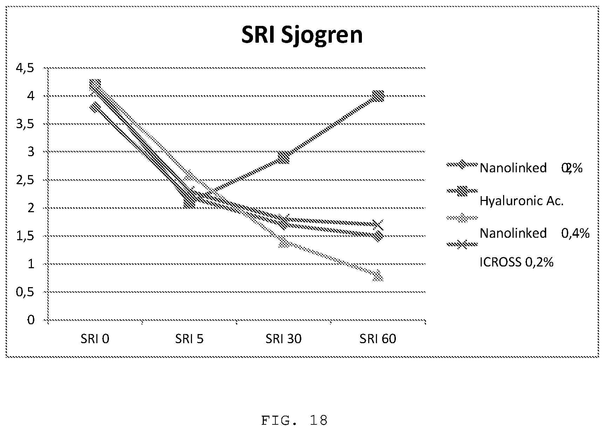

figs. 17 and 18 compare the trend of the SRI parameter (surface regularity index) for the linear hyaluronic acid, nanostructured linked hyaluronic acid at 0.2%, nanostructured linked hyaluronic acid at 0.4 % and hyaluronic acid derivatized with EDC respectively in subjects of a control group without dry eye syndrome, and in subjects with dry eye syndrome associated with Sjogren's syndrome;

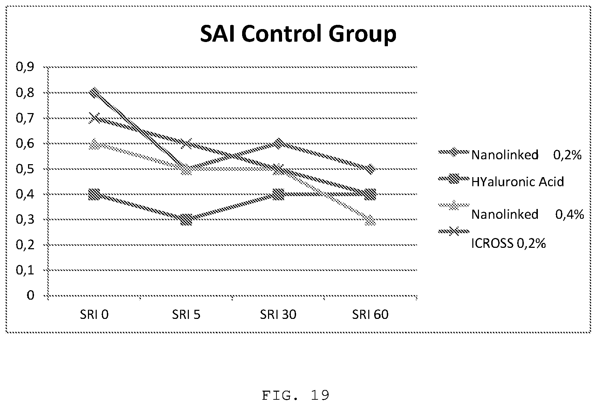

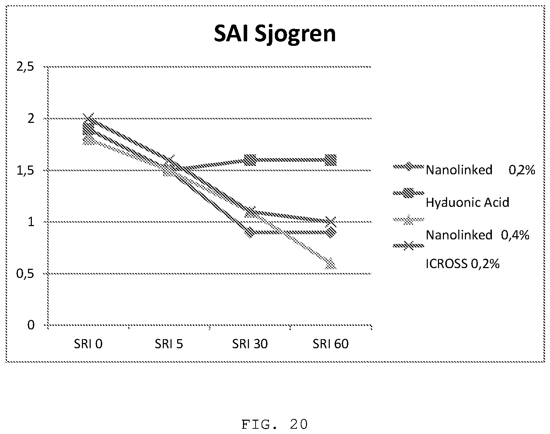

figs. 19 and 20 compare the trend of the SAI parameter (ocular surface asymmetry index) for the linear hyaluronic acid, nanostructured linked hyaluronic acid at 0.2%, nanostructured linked hyaluronic acid at 0.4% and hyaluronic acid derivatized with EDC, respectively in subjects with and without Sjogren's syndrome.

As can be seen, figures 1-12 refer specifically to the analyses carried out on the filterability of pure hyaluronic acid and in aggregate forms and at different concentrations, and are attributable to four different groups:
figures 1 to 4 refer to standard hyaluronic acid which is filterable and therefore stabilizable at 0.2 microns;
figures 5 and 6 refer to cross-linked hyaluronic acid at 10% w/w which, due to its high concentration, is not filterable and therefore not sterilizable at 0.2 microns;
figures 7, 8, 9, 10, 11 and 12 refer to hyaluronic acids cross-linked at a concentration between 4 and 1%, which are filterable and therefore sterilizable at 0.2 microns;
figures 11A and 11B refer to a cross-linked hyaluronic acid at 4% which has not undergone filtration, and which demonstrates the presence (Light Scattering in Volume Mode) at 95% size (particle size) of 10 nanometers.

This indicates that cross-linking at a concentration between 4% and 1% gives the possibility of selecting a nanostructured linked hyaluronic acid which is filterable at 0.2 microns and therefore non-thermally sterilizable.

The characteristics of the invention will be better understood from the detailed description of the experimentation carried out and from the attached figures.

### EXPERIMENTAL ACTIVITY

The experiments began with the preparation of a 10% sodium hyaluronate solution in an alkaline medium.

### FIRST EXPERIMENTAL STEP

### FIRST ATTEMPT

### Point 1: Preparation of hyaluronic acid gel (sodium salt) cross-linked with BDDE

### Step A: Preparation 10% sodium hyaluronate solution in an alkaline medium

1000 mg of hyaluronic acid sodium salt are added to 10 ml of 1 M NaOH.

The hyaluronic acid is completely solubilized.

Time necessary: 1 hour

### Step B: Cross-linking reaction: first day

- 100 mcl of BDDE are added to the viscous gel drop by drop, in portions of 20 mcl each;
- Slow mixing for 2 minutes;
- Leaving at a temperature of 30-31 °C for 6 hours (using a thermostated bath);
- After the 6 hours passed, the formation of a viscous gel was noted;
- The final concentration of the hyaluronic acid sodium salt is 10% during the cross-linking step.

### Step C: Neutralization: first day.

9.15 g of 1 M HCL is added to the viscous gel;
- Mixing for a few minutes;
The final pH is between 5-7;
- The gel is left at room temperature: an increase in the viscosity and compactness of the gel is noted.
- FINAL CONDITIONS:
- 10 ml 1 M NaOH
- 0.1 ml BDDE
- 9.15 ml 1M HCl
- 19.25 gr Final weight

### Step D: Stasis: second and third day

- The gel is left at room temperature (second and third day) for a total of three days; final weight after neutralization: 19.25 gr.

The following were added: 0.57 gr of 1 N HCl to stabilize the pH (6-7); Final weight 19.82 gr.

### Step E: Swelling of the gel fourth and fifth day

- 30.18 g of distilled water are added to reach 50 grams;
- The gel is left in stasis for another two days;
- The gel does not take up all the water available:
   the final weight of the gel after swelling is 25 gr.
- The gel absorbs 25-19.82 = 5.82 gr.

### Step F: Weight of the swollen gel fifth day

The unabsorbed water is removed: about 25 gr;
- the gel is weighed: it weighs 25 grams;
   Swelling: two days
   The gel obtained has a final concentration of hyaluronic acid of 4%: in other words, there is 1 gram of hyaluronic acid per 25 grams of gel.
- From the above, there is clear evidence that an excellent compact gel has been obtained, which at this point can be considered, per se, as an excellent filler.

### Point 2: Final dilution of the gel at 0.4%

- Enough distilled water was added to the gel obtained in **Step F (Point 1)** to reach 250 gr (25 gel + 225 grams of water) to obtain a concentration of 0.4% (1 gr hyaluronic acid/250 gr);
   - Slow mixing for a few minutes;
   - A final volume of 250 ml is obtained with a concentration of 0.4% cross-linked hyaluronic acid, at pH: 5-6.

### Point 3: Micronization of the gel at 0.4% cross-linked hyaluronic acid

- 250 ml of 0.4% product are homogenized with a simple homogenizer such as a kitchen blender or chopper, at maximum power and speed **(from 1000 to 1300 rpm)** for 10 minutes in 2 minute sequences 5 times.

### Point 4: Filtration at 0.2 microns of the homogenized product

- the product homogenized with a common homogenizer, which appears perfectly dispersed and transparent, is filtered at 0.2 microns.

### RESULT

### - Despite the appearance, the product is NOT FILTERABLE.

### - TITER

Hyaluronic acid 0.4% before filtration.

Hyaluronic acid 0.00% after filtration (on the filtrate).

### SECOND ATTEMPT

- In light of the results obtained, steps were taken to:
   - 1) Prepare a hyaluronic acid gel (sodium salt) cross-linked with BDDE;
   - 2) Dilute the gel to a final concentration of **0.2%** instead of 0.4%;
   - 3) Homogenize the gel diluted to **0.2%** using a cutting force produced by a homogenizer;
   - 4) Sterilize the homogenized product by filtration at 0.2 microns.

Following the previously-indicated procedure up to **Point 2,** a gel diluted to 0.2% was obtained.

### Point 3: Micronization of the 0.2% cross-linked hyaluronic acid gel

- 250 ml of 0.2% product are homogenized with a homogenizer at maximum power and speed for 2 minutes, 5 times for a total of 10 minutes;

### Point 4:_Filtration at 0.2 microns of the product thus homogenized

- the product homogenized with a homogenizer, which appears perfectly dispersed and transparent, is filtered at 0.2 microns:
   - **RESULT**
   - **Also in this case, the product is NOT filterable**
   - **TITER**

Hyaluronic acid 0.2% before filtration;
Hyaluronic acid 0.00% after filtration (on the filtrate).

The dimensions of the cross-linked hyaluronic sodium solutions which form the 10% gel once diluted to 0.2% w/w are shown in the analysis by Light Scattering in both Intensity and Volume mode in the graphs in fig. 5 and fig. 6. The results show that the particle size of this hyaluronic acid already cross-linked at 0.2% is greater than 1000 nm in both the Intensity mode and Volume mode analysis.

### CONCLUSION: First experimental step

- A production method of a 4% cross-linked hyaluronic acid gel with a good consistency has been developed,
- using an alkaline condition for 1M NaOH,
- using a 10% hyaluronic acid concentration in the cross-linking reaction,
- using a 10% concentration of BDDE with respect to the concentration of hyaluronic acid (1/10 ratio).
- From the gel obtained, it was not possible to obtain sterilizable solutions by filtration at 0.2 microns, already with a homogenized solution of 0.2% gel and even more so with a homogenized solution of 0.4% gel.

Table 1 below summarizes the results obtained in the two previous attempts, where the characteristics of the non-cross-linked hyaluronic acid used for the cross-linking reaction, at the same concentrations of 0.2 and 0.4%, are indicated in the first two lines. In this Table, and in all the following Tables which will be reported in the present description, the columns, starting from the left, are indicated with letters of the alphabet and represent respectively:
**Column A:** Hyaluronic acid concentration in the cross-linking reaction;
**Column B:** BDDE concentration with respect to the hyaluronic acid concentration;
**Column C:** NaOH molarity: used to solubilize the hyaluronic acid;
**Column D:** Incubation time during the cross-linking reaction;
**Column E:** HCl molarity to neutralize the cross-linking reaction at the end of the incubation;
**Column F:** Dilution % of the cross-linked gel after dilution with distilled water;
**Column G:** Homogenization mode with home blender;
**Column H:** Filterability of the homogenized gel at 0.2 microns;
**Column I:** Titer of the cross-linked product after filtration at 0.2 microns;
**Column L:** pH of the filtered product;
**Column M:** Size, in Intensity mode, of the product subjected to filtration at 0.2 µ;
**Column N:** Size, in Volume mode, of the product subjected to filtration at 0.2 µ.

**Table n. 1**

| | **% crosslinking react** | **BDDE/Hyal. acid weight ratio** | **NaOH** | **Incubation at 30°C** | **HCl** | **% Final Dilution** | **Homogen.** | **0,2 µ Filterability** | **% Filtrate Titer** | **Ph** | **Size nm, Intensity** | **Size nm, Volume** |
|---|---|---|---|---|---|---|---|---|---|---|---|---|
| **Hyaluronic Acid** | **Non-cross-Linked** | **---** | **----** | **----** | **----** | **----** | **----** | **Yes** | **0,4** | **5-6** | **1010** | **0,8-33%** |
| | | | | | | | | | | | | **1,0-33%** |
| | | | | | | | | | | | | **3,5-33%** |
| **Hyaluronic Acid** | **Non-cross-Linked** | **---** | **----** | **----** | **----** | **----** | **----** | **Yes** | **0,2** | **5-6** | **2245** | **0,9047-100%** |
| **Hyaluronic Acid** | **10%** | **1/10** | **1M** | **6 hours** | **1M** | **0,4** | **5 X 2 minutes** | **No** | **0** | **5-6** | | **1998** |
| **Hyaluronic Acid** | **10%** | **1/10** | **1M** | **6 hours** | **1M** | **0,2** | **5 X 2 minutes** | **No** | **0** | **5-6** | **1994** | **4440-55%** |
| | | | | | | | | | | | | **1168-44,6** |

### Dimensional analysis of the hyaluronic acid sodium salt

Fig. 1,2,3 and 4 show for clarity the size of the solutions of hyaluronic acid sodium, (Intensity and Volume distribution in Light Scattering) used for the preparation of the 10% gel in the cross-linking reactions, respectively at 0.4% and 0.2%.

### Interpretation of the particle size analysis of the solutions of hyaluronic acid sodium salt, starting product, before being cross-linked

The particle size of the hyaluronic acid sodium salt at a concentration of 0.4% and 0.2% is greater than 300 nm, as is highlighted by the analysis in INTENSITY MODE where the larger (or aggregate) particles are highlighted even if they are low in number, but filterable in all cases; this means that it is a structure which aggregates in an aqueous solution, is fluid and not rigid and therefore does not prevent the sterilization of the polymer.
- The above-stated is further substantiated by observing the analysis carried out in VOLUME MODE: note that the particle size of the hyaluronic acid is between 0.5 and 2 nm, and this clarifies why the product is very small and therefore filterable.
- Furthermore, it can be said that the analysis in VOLUME MODE is closer to that experimentally obtained from the filtration at 0.2 microns.
- The comparison of the results reported in Table 1 allows to understand the differences in size of the cross-linked hyaluronic acid (after homogenization) at 0.4 and 0.2% with respect to the hyaluronic acid sodium salt at the same concentration of 0.4% and 0.2%.
- It is apparent that the homogenization step, for which simple choppers with a number of revolutions between 2000 and 300 rpm were used, is not sufficient to guarantee filterability starting from an initial concentration of 10% hyaluronic acid in the cross-linking reaction.

### SECOND EXPERIMENTAL STEP

### Procedure for modifying the molecularity of the cross-linking reaction

- The results indicated above show that although the concentration of hyaluronic acid in the cross-linking reaction step is not particularly high (in our case 10%, while normally concentrations of 15, 18, 20, 22, 24, 26, 28% are normally used), the gel thus obtained and then diluted to 0.4 and 0.2% and homogenized with a homogenizer such as a chopper, is not sterilizable by filtration, i.e. it does not pass through the filter.
- This result is opposite to that of the initial hyaluronic acid sodium salt which is easily filtered once diluted to 0.4% and 0.2% with distilled water.

It was therefore decided to prepare hyaluronic acid gels at lower concentrations in the cross-linking step, with the assumption that the BDDE could crosslink the hyaluronic acid molecules in a less comprehensive way.
- The inventors then tried to modify the molecularity of the reaction in order to always have cross-linked but less extensive or better, less compact, hyaluronic acid molecules.
- For this purpose, hyaluronic acid gels were prepared at concentrations of 8 and 60.

The procedure below is along the lines of the previous description.
- The preparation times are the same (6 days);
- The ratio between BDDE and hyaluronic acid is the same 1/10;
- The medium is alkaline for 1 M NaOH.
- The substantial change is in the cross-linking reaction, where the hyaluronic acid has a concentration of less than 10%.

In the tests carried out it was surprisingly found that if a 4% hyaluronic acid dilution is accompanied by a 20% increase in BDDE concentration with respect to the hyaluronic acid quantity by weight, both a good cross-linking and a good filterability are obtained, obtaining a product which is sterilizable at 0.2 microns.

The results shown in the comparative Table 2 below indicate that reducing the concentration of hyaluronic acid in the cross-linking reaction from the initial 10% to 60 leads to a slight improvement in the filterability at 0.2 microns of the 0.2% homogenized solution in distilled water.

**Table n. 2**

| | % Crosslinking react | BDDE/Hyal. acid weight ratio | NaOH | Incubation at 30°C | HCl | **% Final Dilution** | **Homogen.** | **0,2µ Filterability** | **% Filtrate Titer** | **Ph** | **Size nm, Intensity** | **Size nm, Volume** |
|---|---|---|---|---|---|---|---|---|---|---|---|---|
| **Hyaluronic Acid** | **Non-cross-Linked** | **---** | **---** | **---** | **---** | **---** | **---** | **Yes** | **0,4** | **5-6** | **1010** | **0,8-33%** |
| | | | | | | | | | | | | **1,0-33%** |
| | | | | | | | | | | | | **3,5-33%** |
| **Hyaluronic Acid** | **Non-cross-Linked** | **---** | **---** | **---** | **---** | **---** | **---** | **Yes** | **0,2** | **5-6** | **2245** | **0,9047-100%** |
| Hyaluronic Acid | **10%** | **1/10** | **1M** | **6 hours** | **1M** | **0,4** | **5 x 2 minutes** | **No** | **0** | **5-6** | | **1998** |
| Hyaluronic Acid | **10%** | **1/10** | **1M** | **6 hours** | **1M** | **0,2** | **5 x 2 minutes** | **No** | **0** | **5-6** | **1994** | **4440-55%** |
| | | | | | | | | | | | | **1168-44,6** |
| Hyaluronic Acid | **8%** | **1/10** | **1M** | **6 hours** | **1M** | **0,2** | **5 x 2 minutes** | **No** | **0** | **5-6** | **Not determined** | |
| Hyaluronic Acid | **6%** | **1/10** | **1M** | **6 hours** | **1M** | **0,2** | **5 x 2 minutes** | **Very difficult** | Some drops | **5-6** | **Not determined** | |

### THIRD EXPERIMENTAL STEP

### Procedure for optimizing the molecularity of the cross-linking reaction

Continuing with this approach, a product was prepared starting from a concentration **of 4% hyaluronic acid** in the cross-linking reaction instead of the previous concentrations 10%, 8% and 6%.

The results are shown in the following Table 3 and in figures 7 and 8.

**Table n. 3**

| | % Crosslinking react | BDDE/Hyal. acid weight ratio | NaOH | Incubation at 30°C | HCl | **% Final Dilution** | **Homogen.** | **0,2µ Filterability** | **% Filtrate Titer** | **Ph** | **Size nm, Intensity** | **Size nm, Volume** | **Conc BDDE** |
|---|---|---|---|---|---|---|---|---|---|---|---|---|---|
| **Hyaluronic Acid** | **Non-cross-Linked** | --- | --- | --- | --- | --- | --- | **Yes** | **8, 4** | **5-6** | **1010** | **0,8-33%** | |
| | | | | | | | | | | | | **1,0-33%** | |
| | | | | | | | | | | | | **3,5-33%** | |
| **Hyaluronic Acid** | **Non-cross-Linked** | --- | --- | --- | --- | --- | --- | **Yes** | **0,2** | **5-6** | **2245** | **0,9047-100%** | |
| Hyaluronic Acid | **10%** | **1/10** | **1M** | **6 hours** | **1M** | **0,4** | **5 x 2 minutes** | **No** | **0** | **5-6** | | **1998** | |
| Hyaluronic Acid | **10%** | **1/10** | **1M** | **6 hours** | **1M** | **0,2** | **5 x 2 minutes** | **No** | **0** | **5-6** | **1994** | **4440-55%** | |
| | | | | | | | | | | | | **1168-44,6** | |
| Hyaluronic Acid | **8%** | **1/10** | **1M** | **6 hours** | **1M** | **0,2** | **5 x 2 minutes** | **No** | **0** | **5-6** | **Not determined** | | |
| Hyaluronic Acid | **6%** | **1/10** | **1M** | **6 hours** | **1M** | **0,2** | **5 x 2 minutes** | **Very difficult** | **0,01** | **5-6** | **Not determined** | | |
| Hyaluronic Acid | **4%** | **1/5** | **1M** | **6 hours** | **1M** | **0,2** | **5 x 2 minutes** | **Filterable** | **0,165** | **5-6** | **24,85-2740** | **11,68-98%** | **<2ppm** |

### Interpretation of the size analysis of the 0.2% homogenized cross-linked hyaluronic acid solution filtered at 0.2 microns at room temperature

### - Analysis by Light Scattering in Intensity Mode

- As can be seen in the graph in fig. 7, the size of the 0.2% solution of cross-linked hyaluronic acid filtered at 0.2 microns is between 24 and 2740 nm with an average of 430 nm in INTENSITY MODE, where the larger (or aggregate) particles are highlighted, even if low in number.

### - Analysis by Light Scattering in Volume Mode (fig. 8)

- The size of the 0.2% solution of cross-linked hyaluronic acid filtered at 0.2 microns is between 01-9 nm in VOLUME MODE, where the medium and small size particles are highlighted.
- Furthermore, note that in this Volume Mode the presence of particles with a size of less than 5 nm is not shown, which indicates that the initial hyaluronic acid has been cross-linked if not totally, at least in a preponderant manner.

### - EXCELLENT RESULT

### Second Test

Continuing with this approach, a product was prepared starting from a concentration of 10 hyaluronic acid in the cross-linking reaction.

The results are shown in Table 4 below and in graphs 9 and 10.

**Table n. 4**

| | % Crosslinking react | BDDE/Hyal. acid weight ratio | NaOH | Incubation at 30°C | HCl | **% Final Dilution** | **Homogen.** | **0,2µ Filterability** | **% Filtrate Titer** | **Ph** | **Size nm, Intensity** | **Size nm, Volume** | **Conc BDDE** |
|---|---|---|---|---|---|---|---|---|---|---|---|---|---|
| **Hyaluronic Acid** | **Non-cross-Linked** | --- | --- | --- | --- | --- | **---** | **Yes** | **0,4** | **5-6** | **1010** | **0,8-33%** | |
| | | | | | | | | | | | | **1,0-33%** | |
| | | | | | | | | | | | | **3,5-33%** | |
| **Hyaluronic Acid** | **Non-cross-Linked** | --- | --- | --- | --- | --- | **---** | **Yes** | **0,2** | **5-6** | **2245** | **0,9047-100%** | |
| Hyaluronic Acid | **10%** | **1/10** | **1M** | **6 hours** | **1M** | **0,4** | **5 x 2 minutes** | **No** | **0** | **5-6** | | **1998** | |
| Hyaluronic Acid | **10%** | **1/10** | **1M** | **6 hours** | **1M** | **0, 2** | **5 x 2 minutes** | **No** | **0** | **5-6** | **1994** | **4440-55%** | |
| | | | | | | | | | | | | **1168-44,6** | |
| Hyaluronic Acid | **8%** | **1/10** | **1M** | **6 hours** | **1M** | **0,2** | **5 x 2 minutes** | **No** | **0** | **5-6** | **Not determined** | | |
| Hyaluronic Acid | **6%** | **1/10** | **1M** | **6 hours** | **1M** | **0,2** | **5 x 2 minutes** | **Very difficult** | **Some drops** | **5-6** | **Not determined** | | |
| Hyaluronic Acid | **4%** | **1/5** | **1M** | **6 hours** | **1M** | **0,2** | **5 x 2 minutes** | **Filterable** | **0,165** | **5-6** | **24,85-2740** | **11,68-98%** | **<2ppm** |
| Hyaluronic Acid | **1%** | **1/5** | **1M** | **6 hours** | **1M** | **0,2** | **5 x 2 minutes** | **Filterable** | **0,175** | **5-6** | **1275** | **13, 88-96,8** | **<2ppm** |

### INTERPRETATION OF THE SIZE ANALYSIS OF THE SOLUTION WITH 0.2% CROSS-LINKED HYALURONIC ACID HOMOGENIZED AND FILTERED AT 0.2 MICRONS AT ROOM TEMPERATURE

### Analysis by Light Scattering in Intensity Mode

- On the basis of Table 4 and with reference to figures 9 and 10, the size of the 0.2% solution of cross-linked acid filtered at 0.2 microns is between 42 and 844 nm in INTENSITY MODE, where the larger (or aggregate) particles are highlighted, even if low in number.

### - Analysis by Light Scattering in Volume Mode

- The size of the 0.2% solution of cross-linked hyaluronic acid filtered at 0.2 microns is between 13 and 9 nm in Volume Mode, where the medium and small size particles are highlighted.
- Furthermore, note that in this VOLUME MODE the presence of particles with a size of less than 5 nm is not shown, which indicates that the initial hyaluronic acid has been cross-linked if not totally, at least in a preponderant manner.

The preparation of the 1% gel gives good results, but it certainly involves managing larger volumes with respect to the preparation of the 4% gel.

### Results obtained thus far

- Starting from a 4% cross-linking reaction in hyaluronic acid sodium salt in an alkaline medium for 1M NaOH and BDDE at a concentration equal to 20% with respect to that of the hyaluronic acid, a product was obtained in the form of a gel which, suitably diluted with distilled water to 0.2% and homogenized with a chopper, is filterable at 0.2 microns.
- Size indicates a particle distribution around 10-12 nm.
- Starting from a 1% cross-linking reaction in hyaluronic acid sodium salt in an alkaline medium for 1M NaOH and BDDE at a concentration equal to 20% with respect to that of the hyaluronic acid, a product was obtained in the form of a gel which, suitably diluted with distilled water to 0.2% and homogenized with a chopper, is filterable at 0.2 microns.
- Size indicates a particle distribution around 10-12 nm.
- To better understand how the alkalinity can influence the cross-linking reaction, the concentration of sodium hydroxide was decreased, passing from 1M NaOH to 0.2M NaOH.

A test was therefore carried out with 0.2M NaOH in the cross-linking reaction, following the procedure already described. 0.2M HCl was used in the neutralization step.

The results are summarized in the graphs in fig. 11 and 12, 11A and 12A and in the following Table 5.

**Table N. 5**

| | % Crosslinking react | BDDE/Hyal. acid weight ratio | NaOH | Incubation at 30°C | HCl | **% Final Dilution** | **Homogen.** | **0,2µ Filterability** | **% Filtrate Titer** | **Ph** | **Size nm, Intensity** | **Size nm, Volume** | **Cone BDDE** |
|---|---|---|---|---|---|---|---|---|---|---|---|---|---|
| **Hyaluronic Acid** | **Non-cross-Linked** | --- | --- | --- | --- | --- | --- | **Yes** | **0,4** | **5-6** | **1010** | **0,8-33%** | |
| | | | | | | | | | | | | **1,0-33%** | |
| | | | | | | | | | | | | **3,5-33%** | |
| **Hyaluronic Acid** | **Non-cross-Linked** | --- | --- | --- | --- | **---** | **---** | **Yes** | **0,2** | **5-6** | **2245** | **0,9047-100%** | |
| Hyaluronic Acid | **10%** | **1/10** | **1M** | **6 hours** | **1M** | **0,4** | **5 x 2 minutes** | **No** | **0** | **5-6** | | **1998** | |
| Hyaluronic Acid | **10%** | **1/10** | **1M** | **6 hours** | **1M** | **0,2** | **5 x 2 minutes** | **No** | **0** | **5-6** | | **1998** | |
| Hyaluronic Acid | **8** | **1/10** | **1M** | **6 hours** | **1M** | **0,2** | **5 x 2 minutes** | **No** | **0** | **5-6** | **Not determined** | | |
| Hyaluronic Acid | **6** | **1/10** | **1M** | **6 hours** | **1M** | **0,2** | **5 x 2 minutes** | **Very difficult** | **Some drops** | **5-6** | **Not determined** | | |
| Hyaluronic Acid | **4** | **1/5** | **1M** | **6 hours** | **1M** | **0,2** | **5 x 2 minutes** | **Filterable** | **0,165** | **5-6** | **430** | **11,68 -98%** | **<2ppm** |
| Hyaluronic Acid | **1** | **1/5** | **1M** | **6 hours** | **1M** | **0,2** | **5 x 2 minutes** | **Filterable** | **0,170** | **5-6** | **1275** | **13,88 -96,8%** | **<2ppm** |
| Not filtered Hyaluronic Acid | **4** | **1/5** | **0,2M** | **6 hours** | **1M** | **0,2** | **5 x 2 minutes** | | **0,194** | **5-6** | **415** | **10,28-99,0%** | **<2ppm** |
| | | | | | | | | | | | | **110-0,1** | |
| | | | | | | | | | | | | **1217-0,8%** | |
| Filtered Hyaluronic Acid | **4** | **1/5** | **0,2M** | **6 hours** | **1M** | **0,2** | **5 x 2 minutes** | **Filterable** | **0,185** | **5-6** | **22,14-288** | **9,035-9,99%** | **<2ppm** |

### Interpretation of results

The distribution in Intensity Mode indicates that the filtered product has a majority peak (70%) at 22.14 nm while the distribution in Volume Mode has a majority peak at 9.03 nm at a percentage of 95.9%. The same product in figs. 11a and 12a, unfiltered is at 18.3 nm at 44.4% and 135 nm at 10% while in Volume Mode it has a majority peak at 10.28 nm with a percentage of 99.0% and a second peak at 1217nm with 0.8%.

### Conclusions:

- The concentration of sodium hydroxide at 0.2M in the cross-linking reaction of the hyaluronic acid with 4% concentration guarantees very satisfactory polymerization of the hyaluronic acid and also selects a particular range of sizes between 6-12 nm once the 0.2 micron solution has been filtered.
- The above experiments indicated a procedure for preparing cross-linked hyaluronic acid solutions with BDDE which are filterable at 0.2 µ.
- Furthermore, the experiments reported thus far indicate a procedure which identifies/selects a particular population of cross-linked hyaluronic acid with a particular size (particle size) between 8-25 nanometers, i.e. nanostructured.
- This new cross-linked hyaluronic acid can therefore be called nanostructured linked, or for brevity, as already indicated in the present description, "nano-linked."
- According to the present invention, the next step was to transform these solutions (0.2% and 0.4%) of cross-linked hyaluronic acid into eye drops.

### Preparation of NANO-LINKED hyaluronic acid eye drops at a 0.4% concentration which is sterilizable by filtration at 0.2 microns

### a) Preparation of 0.4% NANO-LINKED hyaluronic acid eye drops

The 0.4% cross-linked product solution obtained in the manners described above is transformed into eye drops by following what indicated below:
- 4.45 gr boric acid is added to the 625 ml indicated in point 2 (conc.: real 0.712%, theoretical 0.775%);
- 0.685 gr sodium tetraborate decahydrate is added to the 625 ml indicated in point 2 (real conc. 0.110%, theoretical conc. 0.120%);
- Leaving under stirring for 5 hours;
- pH control: measured value 7.4.
- At this point proceed to:

### b) Preparation of sterile eye drops by filtration at 0.2 microns, through the following steps:

### Homogenization

- 625 ml of the 0.4% cross-linked hyaluronic acid, buffered with borate and isotonic buffer, is homogenized with a chopper for 10 minutes at maximum speed, 5 minutes x2, final temperature 39-40°C.

### Filtration at 0.2 microns

- The eye drops are filtered at 0.2 microns: the eye drops are FILTERABLE and therefore sterilizable.

### Determination of size

Size control (sample with BDDE 4% conc. 0.4%);
Sample taken for osmolality mOsm/kg = 305;
Size analysis 0.4% sterile eye drops;

### Initial concentration hyaluronic acid 4% cross-linking reaction

1.0M NaOH
BDDE concentration 100mg/500mg, 20% with respect to the hyaluronic acid
Diluted to 0.4%; buffered for borate buffer;
Sterilizable product at 0.2 microns;
Filterable product at 0.2 microns;
Titer: BDDE < 2 ppm;
Titer: cross-linked hyaluronic acid 0.350%.

Below is an indicative formula of a nano-linked hyaluronic acid based eye drops at a theoretical value of 0.4%

### 0.4% NANO-LINKED EYE DROP FORMULATION

| **Ingredients** | **% Theoretical concentration** | **Real Concentration %** |
|---|---|---|
| **NANOSTRUCTURED LINKED hyaluronic acid** | **0.400** | **0.375-0.415** |
| **Boric acid** | **0.775** | **0.712** |
| **Sodium decahydrate tetraborate** | **0.120** | **0.110** |
| **NaCl** | **0.560** | **0.450-0.600** |
| **pH** | **7.4** | **7-7.5** |
| **Osmolality mOsm/Kg** | **260-310** | **310** |
| **Sterile by filtration at 0.2 µ** | **YES** | **YES** |
| **Residual BDDE ppm** | **<2** | **Compliant** |
| **Size nm** | **5-25 nm <200** | **Compliant** |

The product is sterilized by filtration.

12 vials are prepared for an in vivo evaluation.

### Preparation of NANO-LINKED hyaluronic acid eye drops at a concentration of 0.2% which is sterilizable by filtration at 0.2 microns

- A 0.2% cross-linked hyaluronic acid solution, obtained as described above, is transformed into eye drops as follows:
   - The 0.2% cross-linked product solution (1250 ml) is transformed into eye drops by following the instructions below:
   - Boric acid is added to the 1250 ml: 9.095 gr (0.742% w/w)
   - Sodium tetraborate decahydrate is added to the 1250 ml: 1.46 g (0.117% w/w)
   - Leaving under stirring for 5 hours;
   - pH control: measured value 7.4.
   - Osmolality mOsm/kg = 224
   - Addition of NaCl: theoretical calculations are carried out to reach the appropriate osmolality.
   - Thus follows the:

### - Preparation of sterile eye drops by filtration at 0.2 µ

### Homogenization

- 1250 ml of 0.2% eye drops of cross-linked hyaluronic acid is homogenized with a chopper for 10 minutes at maximum speed 5 times for 2 minutes. Final temperature 39-40°C
- Filtration is carried out at 0.2 µ.
- The eye drops are filtered at 0.2 µ: the eye drops are **FILTERABLE** and therefore sterilizable.

### Determination of size by Light Scattering

Size analysis 0.2% sterile eye drops;
Initial concentration hyaluronic acid 4% cross-linking reaction 1M NaOH;
BDDE concentration 100mg/500mg, 20% with respect to the weight of the hyaluronic acid;
It is diluted to 0.2% and buffered with isotonic borate buffer;
The product is sterilizable at 0.2 µ
BDDE titer < 2 ppm
Cross-linked hyaluronic acid titer: 0.190%

For purely indicative, non-limiting purposes, a formulation of 0.2% nano-linked hyaluronic acid eye drops is given below:

### FORMULA 0.2% NANO-LINKED ACID EYE DROPS

| **Ingredients** | **Theoretical % concentration** | **Real % concentration** |
|---|---|---|
| **Nanostructured -linked hyaluronic acid** | **0.2** | **0.190** |
| **Boric acid** | **0.775** | **0.742** |
| **Sodium decahydrate tetraborate** | **0.120** | **0.117** |
| **NaCl** | **0.450** | **0.420** |
| **pH** | **7.4** | **7-7.5** |
| **Osmolality mOsm/Kg** | **260-310** | **275** |
| **Sterile by filtration at 0.2 microns** | **yes** | **yes** |
| **Residual BDDE ppm** | **<2** | **Compliant** |
| **Size nm** | **>5 less than 200** | **Compliant** |

Filtration at 0.2 µ: the product is filtered at 0.2 µ:
the product is **FILTERABLE.**
Size control (sample with 4% BDDE, conc. 0.2%);
Filterable product at 0.2 µ;
pH: 7.13. Osmolality value mOsm/Kg = 224

After adding NaCl osmolality value mOsM/kg 275; the product is then sterilized by filtration, 14 vials are prepared for in vivo evaluation.

### Preparation of 0.4% eye drops in TRIS buffer. TRIS HCl

Cross-linked hyaluronic acid (sodium salt) is prepared for an ophthalmic solution at an initial hyaluronic acid concentration of 4%.

BDDE/Hyaluronic acid sodium salt ratio 1/5 (100 mg/500 mg) (new hyaluronic acid).

Preparation of the product in TRIS buffer.
- 0.600 g Tris HCl is added for a final TRIS HCl concentration of 0.48% w/w
- Tris 0.060 gr is added for a final TRIS concentration of 0.048% w/w
- Leaving under stirring for 5 minutes
- Control pH at 7.45-7.5
- pH value detected: 7.45
- Osmolality mOsm/Kg = 255

### Point 6: Homogenization

- 125 ml of the 0.4% product is homogenized with a chopper for 10 minutes at maximum speed 5 minutes x 2, final temperature 39-40°C.

### Point 7: Filtration at 0.2 microns:

the product is filtered at 0.2 microns, and is **FILTERABLE.**

### Point 8: Determination of size

BDDE concentration 100mg/500mg, 20% with respect to the hyaluronic acid
Diluted to 0.4% buffered for TRIS buffer
Sterilizable product 0.2 microns
BDDE titer < 2 ppm
Osmolality value mOsm/kg = 255
Tris/Tris H7Cl buffer, pH 7.47
Filterable product at 0.2 microns

### 0.4% NANO-LINKED ACID EYE DROP FORMULATION IN TRIS-TRIS HCL BUFFER

| Ingredients | Theoretical % concentration | Real Concentration % |
|---|---|---|
| Nanostructuredlinked hyaluronic acid | 0.4 | 0.385 |
| TRIS HCl | 0.480 | |
| TRIS | 0.048 | |
| NaCl | 0.450 | 0.460 |
| pH | 7-7.5 | 7.45 |
| Osmolality mOsm/Kg | 260-310 | 260 |
| Sterile by filtration at 0.2 microns | yes | Yes |
| Residual BDDE ppm | <2 | Compliant |
| Size nm | >5 < 200 | Compliant |

### Preparation of 0.2% eye drops in TRIS buffer. TRIS HCl

The previous preparation is repeated by varying only the final dilution which instead of being 125 ml is 250 ml in order to allow a final concentration of 0.2% w/w nano-linked acid. The appropriate quantities of sufficient tris buffer and sodium chloride are added to these 250 ml to reach a pH between 7 and 7.5 and an osmolality between 260 and 310 milliosmoles/kg.

The product is subjected to filtration at 0.2 µ: the product is FILTERABLE
Sterilizable product 0.2 microns
BDDE titer < 2 ppm

### 0.2% NANO-LINKED ACID EYE DROP FORMULATION IN TRIS-TRIS HCL BUFFER

| Ingredients | Theoretical % concentration | Real concentration % |
|---|---|---|
| Nanostructured linked hyaluronic acid | 0.2 | 0.185 |
| TRIS HCl | 0.480 | 0.480 |
| TRIS | 0.048 | 0.480 |
| NaCl | 0.450 | 0.460 |
| pH | 7-7.5 | 7.45 |
| Osmolality mOsm/Kg | 260-310 | 265 |
| Sterile by filtration at 0.2 microns | Yes | Yes |
| Residual BDDE ppm | <2 | Compliant |
| Size nm | >5 < 200 | Compliant |

Table 6 below summarizes the data related to the buffered eye drops previously obtained.

**Table n. 6**

| | % Crosslinking react | BDDE/Hyal. acid weight ratio | NaOH | Incubation at 30°C | HCl | **% Final Dilution** | **Homogen.** | **0,2µ Filterability** | **% Filtrate Titer** | **Ph** | **Size nm, Intensity** | **Size nm, Volume** | **Cone BDDE** |
|---|---|---|---|---|---|---|---|---|---|---|---|---|---|
| **Hyaluronic Acid** | **Non-cross-Linked** | --- | --- | --- | --- | **---** | --- | **Yes** | **0, 4** | **5-6** | **1010** | **0,8-33%** | |
| | | | | | | | | | | | | **1,0-33%** | |
| | | | | | | | | | | | | **3,5-33%** | |
| **Hyaluronic Acid** | **Non-cross-Linked** | --- | --- | --- | --- | --- | --- | **Yes** | **0,2** | **5-6** | **2245** | **0,9047-100%** | |
| Hyaluronic Acid | **10%** | **1/10** | **1M** | **6 hours** | **1M** | **0,4** | **5 x 2 minutes** | **No** | **0** | **5-6** | | **1998** | |
| Hyaluronic Acid | **10%** | **1/10** | **1M** | **6 hours** | **1M** | **0,2** | **5 x 2 minutes** | **No** | **0** | **5-6** | | **1998** | |
| Hyaluronic Acid | **8** | **1/10** | **1M** | **6 hours** | **1M** | **0,2** | **5 x 2 minutes** | **No** | **0** | **5-6** | **Not determined** | | |
| Hyaluronic Acid | **6** | **1/10** | **1M** | **6 hours** | **1M** | **0,2** | **5 x 2 minutes** | **Very difficult** | **Some drops** | **5-6** | **Not determined** | | |
| Hyaluronic Acid | **4** | **1/5** | **1M** | **6 hours** | **1M** | **0,2** | **5 x 2 minutes** | **Filterable** | **0,165** | **5-6** | **430** | **11,68 -98%** | |
| Hyaluronic Acid | **1** | **1/5** | **1M** | **6 hours** | **1M** | **0,2** | **5 x 2 minutes** | **Filterable** | **0,170** | **5-6** | **1275** | **13,88 -96,8** | |
| **Not filtered Hyaluronic Acid** | **4** | **1/5** | **0,2M** | **6 hours** | **1M** | **0,2** | **5 x 2 minutes** | **Filterable** | **0,194** | **5-6** | **415** | **10,28-99,0%** | |
| | | | | | | | | | | | | **110-0,1** | |
| | | | | | | | | | | | | **1217-0,8%** | |
| **Filtered Hyaluronic Acid** | **4** | **1/5** | **0,2M** | **6 hours** | **1M** | **0,2** | **5 x 2 minutes** | **Filterable** | **0,185** | **5-6** | **42,,67** | **9,035-9,99%** | |
| **NANO-LINKED EYE DROPS, borate Buff.** | **4** | **1/5** | **1M** | **6 hours** | **1M** | **0,4** | **5 x 2 minutes** | **Filterable** | **0,385** | **5-6** | **46, 52** | **13,57-99%** | **<2ppm** |
| | | | | | | | | | | | | **76, 13-0, 2%** | |
| **NANO-LINKED EYE DROPS, borate Buff.** | **4** | **1/5** | **1M** | **6 hours** | **1M** | **0,2** | **5 x 2** | **Filterable** | **0,188** | **5-6** | **31, 99** | **18,23-99%** | **<2ppm** |
| | | | | | | | **minutes** | | | | | **163-0,5%** | |
| **NANO-LINKED EYE DROPS, TRIS Buff.** | **4** | **1/5** | **1M** | **6 hours** | **1M** | **0,4** | **5 x 2 minutes** | **Filterable** | **0,386** | **5-6** | | | **<2ppm** |
| **NANO-LINKED EYE DROPS, TRIS Buff.** | **4** | **1/5** | **1M** | **6 hours** | **1M** | **0,2** | **5 x 2 minutes** | **Filterable** | **8,187** | **5-6** | | | **2ppm** |

### USE OF 0.2% w/w NANO-LINKED EYE DROPS IN BORATE BUFFER IN THE TREATMENT OF DRY EYE SYNDROME.

According to another aspect of the present invention, the nano-linked (nanostructured) hyaluronic acid eye drops, obtained as described, have shown an excellent stabilizing ability of the lacrimal film, both in terms of hyaluronic acid as such, and hyaluronic acid derivatized with EDC, known with the commercial name of "Icross".

The nano-linked hyaluronic acid based eye drops of the invention therefore find specific use in the treatment of dry eye syndrome, as shown below.

Dry eye syndrome is a multifactorial disease of the lacrimal film and of the ocular surface with a prevalence which can vary between 10 and 35% based on the different ocular evaluation methods applied.

Dry eye syndrome is one of the main clinical manifestations of Sjogren's syndrome (SS), a chronic inflammatory disease with an autoimmune basis. Dry eye syndrome can be classified as dry eyes related to Sjogren's syndrome (SSDE) or dry eyes unrelated to Sjogren's syndrome (NSSDE). In any case, the vicious circle which develops on the ocular surface causes inflammation and the latter in turn leads to further dysfunctions of the cells responsible for lacrimal secretion. The standard therapy for any dry eye syndrome is based on the use of artificial tears which increase the volume of the lacrimal filament, integrating it and increasing the stability thereof. The formulations based on hyaluronic acid (HA) are among those which have greater efficacy thanks to the rheological characteristics of the same, the latter being a natural and therefore biocompatible polymer. HA is metabolized in the body by hyaluronidase which effectively reduces the availability thereof. To slow down the physiological degradation process, the HA was modified with EDC, generating the so-called hyaluronic acid ICROSS.

It has already been shown that this new formulation extends the contact time between HA and the ocular surface and previous studies both in animals and in vivo in humans have shown that the use of ICROSS significantly improves the ocular surface and is more effective than the non-cross-linked counterpart.

A prospective randomized study involving patients over 18 years of age led to an analysis of the data concerning the SRI parameter (surface regularity index), i.e. the regularity index of the corneal surface in patients with Sjogren's syndrome and the SAI (the corneal surface asymmetry index) which revealed statistically significant differences between linear hyaluronic acid, (HA), hyaluronic acid (ICROSS), and nano-linked hyaluronic acid, at 0.2% and 0.4%, which is the subject of the present application, the structure of which, determined with Light Scattering (in Volume Mode), has a size of less than 100 nm, as has been shown.

The significance of the results was assessed using the Kolmogorov-Smirnov test to determine the normal distribution of the data. Based on the result, the t test and the Wilcoxon test were used for data coupling. The significance level was found to be P <0.05, which is an excellent result.

### USE TESTS OF NANO-LINKED EYE DROPS IN THE TREATMENT OF DRY EYE SYNDROME

Below are some specifics of the experiments carried out by the experimenters to date.

Corneal topography, with the recording of surface regularity indices, can be used to evaluate the influence of artificial drops on these parameters.

The indices used are:
SAI (surface asymmetry index)
SRI (surface regularity index)

SAI is the asymmetry index of the corneal surface and is calculated through the specular correspondence between the two half-corneas, which normally does not surpass small differences. The topographer compares many adjacent points of a half-cornea with corresponding points of its specular cornea. The index of the ideal sphero-cylindrical surface is 0. Values greater than 1 indicate a considerable reduction in the quality of visual acuity (keratoconus, decentralized refractive surgery, transplantation, deformation).

SRI is the regularity index of the corneal surface and is the result of the comparison between the variations of many adjacent points of the 10 central rings. This area is equivalent to a papilla of about 4.5 cm in diameter. Values greater than 1.5 express irregularities in the optical zone.

This prospective, randomized trial involved patients over the age of 18 with a diagnosis of SSDE. The inclusion criteria for this study are the absence of other eye conditions.

Eye drops containing nano-linked hyaluronic acid at 0.2 and 0.4% w/w respectively were evaluated in vivo for the two parameters SRI and SAI, together with hyaluronic acid derivatized with EDC and linear hyaluronic acid (HA).

Four groups were used for the four treatments consisting of:
10 healthy patients for the positive control and 10 patients with Sjogren's syndrome for the SRI parameter;
10 healthy patients for the positive control and 10 patients with Sjogren's syndrome for the SAI parameter.

The corneal topography was performed 3 consecutive times by an expert in both eyes (time 0) and the average value was calculated for the definitive analysis of the data. A second operator then administered 1 drop of 0.20% ICROSS to 10 control patients and 10 patients with SSDE. 0.2% nano-linked hyaluronic acid was administered to another 10 control patients and 10 patients with SSDE as well as 0.4% nano-linked hyaluronic acid in 10 other control patients and 10 patients with SSDE. 0.29% HA was administered to 10 additional control patients and 10 patients with SSDE. Corneal topography was performed again after 5 minutes (time 1), 30 minutes (time 2), and 60 minutes (time 3) from the administration of the eye drops.

The results are shown in figs. 17-20 and in Tables 7, 8, 9 and 10 below.

**TABLE 7**

| **SRI Control Group** | **Nano-linked 0,2%** | **Ialuronic Ac.** | **Nano-linked 0,4%** | **ICROSS 0,2%** |
|---|---|---|---|---|
| **SRI 0** | **2,5 ± 0,04** | **2,4 ± 0,04** | **2,2 ± 0,08** | **2,1 ± 0.04** |
| **SRI 5** | **2,1 ± 0,12** | **2,1 ± 0,03** | **1,6 ± 0,09** | **1,8 ± 0.07** |
| **SRI 30** | **1,8 ± 0,07** | **2,3 ± 0,02** | **1,5 ± 0,10** | **1,6 ± 0.01** |
| **SRI 60** | **0,8 ± 0,06** | **2,3 ± 0,01** | **0,5 ± 0,11** | **1,6 ± 0,01** |

**TABLE 8**

| **SRI Sjogren** | **Nano-linked 0,2%** | **Ialuronic Ac.** | **Nano-linked 0,4%** | **ICROSS 0,2%** |
|---|---|---|---|---|
| **SRI 0** | **3,8 ± 0,06** | **4,2 ± 0,10** | **4,2 ± 0,02** | **4,1 ± 0,06** |
| **SRI 5** | **2,2 ± 0,05** | **2,1 ± 0,11** | **2,6 ± 0,02** | **2,3 ± 0,04** |
| **SRI 30** | **1,7 ± 0,04** | **2,9 ± 0,02** | **1,4 ± 0,01** | **1,8 ± 0.03** |
| **SRI 60** | **1,5 ± 0,04** | **4 ± 0,03** | **0,8 ± 0,08** | **1,7 ± 0,05** |

**TABLE 9**

| **SAI Control Group** | **Nano-linked 0,2%** | **Ialuronic Ac.** | **Nano-linked 0,4%** | **ICROSS 0,2%** |
|---|---|---|---|---|
| **0** | **0,8 ± 0,07** | **0,4 ± 0,04** | **0,6 ± 0,05** | **0,7 ± 0,06** |
| **5** | **0,5 ± 0,06** | **0,3 ± 0,02** | **0,5 ± 0,10** | **0,6 ± 0,04** |
| **30** | **0,6 ± 0,08** | **0,4 ± 0,02** | **0,5 ± 0,30** | **0,5 ± 0,04** |
| **60** | **0,5 ± 0,06** | **0,4 ± 0,06** | **0,3 ± 0,04** | **0,4 ± 0,01** |

**TABLE 10**

| **SAI Sjogren** | **Nano-linked 0,2%** | **Ialuronic Ac.** | **Nano-linked 0,4%** | **ICROSS 0,2%** |
|---|---|---|---|---|
| **0** | **1,8 ± 0,03** | **1,9 ± 0,08** | **1,8 ± 0,04** | **2 ± 0,11** |
| **5** | **1,5 ± 0,05** | **1,5 ± 0,061** | **1,5 ± 0,07** | **1,6 ± 0,01** |
| **30** | **0,9 ± 0,05** | **1,6 ± 0,10** | **1,1 ± 0,05** | **1,1 ± 0.01** |
| **60** | **0,9 ± 0,06** | **1,6 ± 0,05** | **0,6 ± 0,07** | **1 ± 0,02** |

Already from the controls it is noted that 0.2% nano-linked hyaluronic acid and 0.4% nano-linked hyaluronic acid are better than hyaluronic acid derivatized with EDC and linear hyaluronic acid as regards the SRI parameter (longer permanence) in the control groups.

As can be seen in fig. 17, the statistical analysis of the data concerning the SRI parameter in the control group highlights statistically significant differences between the 0.2% and 0.4% nano-linked hyaluronic acid compared to the linear hyaluronic acid H, and the hyaluronic acid derivatized with EDC.

Therefore, in subjects without dry eye syndrome (controls), the 0.2% and 0.4% nano-linked hyaluronic acids remain longer than the other hyaluronic acids examined.

The same thing also applies to the data concerning the SRI parameter, shown in fig. 18, where it is apparent that statistically significant differences exist between subjects with dry eye syndrome associated with Sjogren's syndrome as regards the regularity index of the corneal surface in the use of the nano-linked hyaluronic acids of the present invention and the use of ICROSS hyaluronic acids already on the market.

Therefore, in subjects with dry eye syndrome associated with Sjogren's syndrome, the two nano-linked acids according to the present invention have a better efficacy than the hyaluronic acids currently on the market.

## Claims

1. A method for preparing cross-linked hyaluronic acid which is filterable at 0.2 µ, **characterized in that** it includes the following steps:
a) performing a cross-linking reaction between a hyaluronic acid sodium salt solution in a basic medium in a concentration between 1% and 4% by weight with respect to the total weight of the composition and BDDE in a 20% concentration with respect to the weight of the hyaluronic acid, so as to obtain a gel where the final concentration of the cross-linked hyaluronic acid sodium salt is between 4% and 1% by weight with respect to the total weight of said gel;
b) partially neutralizing the gel with an acid, preferably HCl, to bring it to a pH between 5.5 and 6.5, to a final concentration of cross-linked hyaluronic acid from 2% to 0.5% w/w, which is equal to half of the initial concentration of hyaluronic acid;
c) allowing the gel to rest at room temperature for a period of time of at least 6 days so as to allow the complete cross-linking of the BDDE and the complete degradation thereof, where the particle size of the cross-linked hyaluronic acid is between 8 - 50 nm;
d) diluting to a concentration between 0.4% and 0.1% w/w said partially neutralized gel with distilled water.

2. A method for preparing cross-linked hyaluronic acid which is filterable at 0.2 µ according to the preceding claim, **characterized in that** in step a) the alkalinity is between 1M and 0.2M in NaOH and the subsequent partial neutralization in step b) takes place with HCl between 1M and 0.2M.

3. A method for preparing an ophthalmic product with cross-linked hyaluronic acid sterilized by filtration at 0.2 µ, **characterized in that** it includes:
preparing said product in borate buffer by adding boric acid and sodium decahydrate tetraborate to across-linked hyaluronic acid obtained according to claim 1 or claim 2 under stirring to a final pH between 7 and 7.4;
homogenizing the product at 0.4% w/w with a blade homogenizer for 10 minutes at a speed between 1000 and 1300 rpm, until reaching a final temperature of 39-40°C;
and
filtering at 0.2 microns the homogenized product for the sterilization thereof.

4. A method for preparing an ophthalmic product which is sterilizable by filtration at 0.2 µ, according to claim 3, **characterized in that** it includes the following steps:
**Step 1:** A solution of 4% sodium hyaluronate is prepared, at a NaOH concentration between 1 and 0.2M;
**Step 2:** An amount by weight of BDDE equal to 20% of hyaluronic acid sodium salt is added to the solution prepared in step 1;
- Mixing for 2 minutes;
- Leaving at a temperature of 30°C for 4-8 hours, preferably 6 hours, thus obtaining a final concentration of hyaluronic acid sodium salt of 4% w/w of the total solution;
**Step 3:** The required amount of 1N HCl is added to bring the gel to a pH between 5.5-6.5, so that the final concentration of the cross-linked hyaluronic acid gel is 2% by weight with respect to the total weight of the final product, optionally adding water;
**Step 4:** The thus neutralized product in the form of a gel is left at room temperature for a sufficient time to completely degrade the possibly present BDDE;
**Step 5:** The gel neutralized in step 3 is diluted with distilled water to obtain the concentration of 0.40%;
- where the pH is between 5.5-6.5 and the osmolality mOsm/Kg is between 145-155.
**Step 6:** To the 0.4% diluted product, boric acid, and sodium decahydrate tetraborate are added as follows:
- Boric Acid: in a sufficient amount to obtain a final concentration in the product between 0.8-0.7%;
- Sodium Tetraborate: in a sufficient amount to have a final concentration of 0.100%;
- Leaving under stirring for 5 hours;
- pH control: 7-7.4;
- Osmolality mOsm/Kg 270-310;
**Step 7:** The 0.4% product is homogenized with a blade homogenizer for 10 minutes at a speed of 1000-1300 rpm, 5 minutes x2, final temperature 39-40°C;
**Step 8:** The product homogenized in step 7 is filtered with a 0.2-micron filter; and
the product is thus sterilized, the pH being between 7-7.5 and osmolality mOsm/kg: 260-310.

5. Sterile eye drops obtained by filtration at 0.2 microns based on nanostructured linked hyaluronic acid prepared according to any of claims 1-4, having the following composition:
| Composition | Theoretical % concentration | Real % concentration |
|---|---|---|
| Nanostructured linked hyaluronic acid | 0.400 | 0.375 -0.415 |
| Boric acid | 0.775 | 0.712 |
| Sodium tetraborate | 0.120 | 0.110 |
| NaCl | 0.560 | 0.450 - 0.600 |
| Sterile by filtration at 0.2 microns | Yes | Yes |
| Osmolality mOsm/kg | 270-310 | 310 |
| Size | 5-less than 20 nm | 2C |
| pH | 7.4 | 7-7.5 |
| BDDE ppm | <2 | Compliant |

6. Sterile eye drops obtained by filtration based on nanostructured linked hyaluronic acid prepared according to any of claims 1-4 with 0.2% BDDE, in borate buffer, having the following composition:
| Ingredients | Theoretical % concentration | Real % concentration |
|---|---|---|
| Nanostructured linked hyaluronic acid | 0.2 | 0.190 |
| Boric acid | 0.775 | 0.742 |
| Sodium decahydrate tetraborate | 0.120 | 0.117 |
| NaCl | 0.450 | 0.420 |
| pH | 7.4 | 7-7.5 |
| Osmolality mOsm/Kg | 260-310 | 275 |
| Sterile by filtration at 0.2 µ | YES | YES |
| Residual BDDE ppm | <2 | Compliant |
| Size nm | 5-25 nm <200 | Compliant |

7. Sterile eye drops obtained by filtration based on 0.4% nanostructured linked acid prepared according to any of claims 1-4 in Tris-Tris HCl buffer having the following composition:
| Ingredients | Theoretical % concentration | Real % concentration |
|---|---|---|
| Nanostructured linked hyaluronic acid | 0.4 | 0.385 |
| TRIS HCl | 0.480 | |
| TRIS | 0.048 | |
| NaCl | 0.450 | 0.460 |
| pH | 7-7.5 | 7.45 |
| Osmolality mOsm/Kg | 260-310 | 260 |
| Sterile by filtration at 0.2 microns | Yes | Yes |
| Residual BDDE ppm | <2 | Compliant |
| Size nm | >5 < 200 | Compliant |

8. Sterile eye drops obtained by filtration based on 0.2% nanostructured linked acid prepared according to any of claims 1-4 in Tris Tris-HCl buffer having the following composition:
| Ingredients | Theoretical % concentration | Real % concentration |
|---|---|---|
| Nanostructured linked hyaluronic acid | 0.2 | 0.185 |
| TRIS HCl | 0.480 | 0.480 |
| TRIS | 0.048 | 0.480 |
| NaCl | 0.450 | 0.460 |
| pH | 7-7.5 | 7.45 |
| Osmolality mOsm/Kg | 260-310 | 265 |
| Sterile by filtration at 0.2 microns | Yes | Yes |
| Residual BDDE ppm | <2 | Compliant |
| Size nm | >5 < 200 | Compliant |

9. Sterile eye drops obtained by filtration at 0.2 microns based on 0.4% w/w nanostructured linked hyaluronic acid in borate buffer according to claim 6, for medical use.

10. Sterile eye drops obtained by filtration at 0.2 microns based on 0.4% w/w nanostructured linked hyaluronic acid in borate buffer according to claim 6, for use in a method for the treatment of dry eye syndrome.

11. Sterile eye drops obtained by filtration at 0.2 microns based on 0.2% w/w nanostructured linked hyaluronic acid in borate buffer according to claim 6 for medical use.

12. Sterile eye drops obtained by filtration at 0.2 microns based on 0.2% w/w nanostructured linked hyaluronic acid in borate buffer according to claim 6 for use in a method for the treatment of dry eye syndrome.

13. Sterile eye drops obtained by filtration at 0.2 microns based on 0.2% w/w nanostructured linked hyaluronic acid in Tris Tris-HCl buffer according to claim 8 for medical use.

14. Sterile eye drops obtained by filtration at 0.2 microns based on 0.2% w/w nanostructured linked hyaluronic acid in Tris Tris-HCl buffer according to claim 8 for use in a method for the treatment of dry eye syndrome.

## Patentansprüche

1. Verfahren zur Herstellung vernetzter Hyaluronsäure, welche bei 0,2 µ gefiltert werden kann, **gekennzeichnet dadurch, dass** es folgende Schritte aufweist:
a) Durchführen einer Vernetzungsreaktion zwischen einer Hyaluronsäure-Natrium-Sal z-Lösung in einem basischen Medium mit einer Konzentration zwischen 1% und 4% nach Gewicht in Bezug auf das Gesamtgewicht der Zusammensetzung mit BDDE mit einer 20% Konzentration in Bezug auf das Gewicht der Hyaluronsäure, um ein Gel zu erhalten, wobei die finale Konzentration des vernetzten Hyaluronsäure-Natrium-Salzes zwischen 4% und 1% nach Gewicht mit Bezug auf das Gesamtgewicht des Gels ist;
b) teilweises Neutralisieren des Gels mit einer Säure, vorzugsweise HCl, um es auf einen pH-Wert zwischen 5,5 und 6,5, auf eine finalen Konzentration der vernetzten Hyaluronsäure von 2% bis 0,5% w/w, welche gleich der Hälfte der ursprünglichen Konzentration der Hyaluronsäure ist, zu bringen;
c) Ermöglichen, dass das Gel, für einen Zeitraum von mindestens 6 Tagen bei Zimmertemperatur ruht, um eine komplette Vernetzung des BDDE und eine vollständige Zersetzung desselben zu ermöglichen, wobei die Teilchengröße der vernetzten Hyaluronsäure zwischen 8 - 50 nm beträgt;
d) Verdünnen des teilweise neutralisierten Gels mit destilliertem Wasser auf eine Konzentration zwischen 0,4% und 0,1% w/w.

2. Verfahren zur Herstellung vernetzter Hyaluronsäure, welche bei 0,2 µ gefiltert werden kann, nach dem vorhergehenden Anspruch, **dadurch gekennzeichnet, dass** in Schritt a) die Alkalität zwischen 1M und 0,2M in NaOH liegt und die folgende teilweise Neutralisation in Schritt b) mit HCl zwischen 1M und 0,2M stattfindet.

3. Verfahren zur Herstellung eines Augenheilprodukts mit vernetzter Hyaluronsäure, welches durch Filtration bei 0,2 µ sterilisiert wird, **dadurch gekennzeichnet, dass** es aufweist:
Herstellen des Produkts in einem Boratpuffer durch Hinzufügen von Borsäure und Natrium-Decahydrat-Tetraborat zu vernetzter Hyaluronsäure, welche gemäß Anspruch 1 oder Anspruch 2 gewonnen wurde, unter Rühren zu einem finalen pH-Wert zwischen 7 und 7,4;
Homogenisieren des Products bei 0,4% w/w mit einem Klingenhomogenisierer für 10 Minuten bei einer Geschwindigkeit zwischen 1000 und 1300 rpm, bis zum Erreichen einer finalen Temperatur von 39-40°C;
und
Filtrieren des homogenisierten Produkts zu dessen Sterilisation bei 0,2 Mikrons.

4. Verfahren zur Herstellung eines Augenheilprodukts, welches durch Filtrieren bei 0,2 µ sterilisiert werden kann, nach Anspruch 3, **dadurch gekennzeichnet, dass** es die folgenden Schritte aufweist:
**Schritt 1:** Eine Lösung von 4% Natrium-Hyaluronat wird bei einer NaOH-Konzentration zwischen 1 und 0,2 M hergestellt;
**Schritt 2:** Eine Menge nach Gewicht von BDDE gleich 20% des Hyaluronsäure-Natrium-Salzes wird zu der in Schritt 1 hergestellten Lösung hinzugefügt;
- Mischen für 2 Minuten;
- Ruhen lassen bei einer Temperatur von 40°C für 4-8 Stunden, vorzugsweise 6 Stunden, um dadurch eine finale Konzentration des Hyaluronsäure-Natrium-Salzes von 4% w/w der gesamten Lösung zu erhalten;
**Schritt 3:** Die benötigte Menge von 1N HCl wird hinzugefügt um das Gel auf einen pH-Wert zwischen 5,5-6,5 zu bringen, so dass die finale Konzentration des vernetzten Hyaluronsäure-Gels 2% nach Gewicht in Bezug auf das Gesamtgewicht des finalen Produkts beträgt, optionales Hinzufügen von Wasser;
**Schritt 4:** Das hierdurch neutralisierte Produkt in Form eines Gels wird für eine ausreichende Zeit bei Raumtemperatur gehalten um möglicherweise vorhandenes BDDE vollständig zu zersetzen;
**Schritt 5:** Das in Schritt 3 neutralisierte Gel wird mit destilliertem Wasser verdünnt, um eine Konzentration von 0,40% zu erhalten;
- wobei der pH-Werte zwischen 5,5-6,5 liegt und die Osmolalität zwischen mOsm/Kg 145-155 liegt;
**Schritt 6:** Zu dem 0,4% verdünnten Produkt werden Borsäure und Natrium-Decahydrat-Tetrabotat wie folgt hinzugefügt:
- Borsäure: in einer ausreichenden Menge um eine finale Konzentration des Produkts zwischen 0,8-0,7% zu erreichen;
- Natrium-Tetraborat: in einer ausreichenden Menge um eine finale Konzentration von 0,100% zu erlangen;
- Rühren für 5 Stunden;
- pH Kontrolle: 7-7,4;
- Osmolalität mOsm/Kg 270-310;
**Schritt 7:** Das 0,4% Produkt wird mit einem Klingenhomogenisierer für 10 Minuten bei einer Geschwindigkeit von 1000-1300 rpm homogenisiert, 5 Minuten x2, finale Temperatur 39-40°C;
**Schritt 8:** Das in Schritt 7 homogenisierte Produkt wird mit einem 0,2-Mikron Filter filtriert; und
das Produkt wird dadurch sterilisiert, wobei der pH-Wert zwischen 7-7,5 und die Osmolalität zwischen mOsm/kg 260-310 liegt.

5. Sterile Augentropfen, welche basierend auf nanostrukturierter vernetzter Hyaluronsäure, hergestellt nach einem der Ansprüche 1-4, durch filtrieren bei 0,2 Mikrons gewonnen werden, mit folgender Zusammensetzung:
| Zusammensetzung | Theoretische % Konzentration | Reale % Konzentration |
|---|---|---|
| Nanostrukturierte vernetzte Hyaluronsäure | 0,400 | 0,375-0,415 |
| Borsäure | 0,7775 | 0,712 |
| Natrium-Tetraborat | 0,120 | 0,110 |
| NaCl | 0,560 | 0,450 - 0,600 |
| Steril durch Filtrierung bei 0,2 Mikrons | Ja | Ja |
| Osmolalität mOsm/kg | 270-310 | 310 |
| Größe | 5-weniger als 20 nm | |
| pH | 7,4 | 7-7,5 |
| BDDE ppm | <2 | übereinstimmend |

6. Sterile Augentropfen, welche basierend auf nanostrukturierter vernetzter Hyaluronsäure, hergestellt nach einem der Ansprüche 1-4, mit 0,2% BDDE, in einem Boratpuffer, durch filtrieren bei 0,2 Mikrons gewonnen werden, mit folgender Zusammensetzung:
| Inhaltsstoffe | Theoretische % Konzentration | Reale % Konzentration |
|---|---|---|
| Nanostrukturierte vernetzte Hyaluronsäure | 0,2 | 0,190 |
| Borsäure | 0,775 | 0,742 |
| Natrium-Decahydrat-Tetraborat | 0,120 | 0,117 |
| NaCl | 0,450 | 0,420 |
| pH | 7,4 | 7-7,5 |
| Osmolalität mOsm/Kg | 260-310 | 275 |
| Steril durch filtrieren bei 0,2 µ | JA | JA |
| Rest-BDDE ppm | <2 | Übereinstimmend |
| Größe nm | 5-25 nm <200 | Übereinstimmend |

7. Sterile Augentropfen, welche basierend auf 0,4% nanostrukturierter vernetzter Säure, hergestellt nach einem der Ansprüche 1-4 in einem Tris-Tris HCl Puffer, durch filtrieren gewonnen werden, mit folgender Zusammensetzung:
| Inhaltsstoffe | Theoretische % Konzentration | Reale % Konzentration |
|---|---|---|
| Nanostrukturierte vernetzte Hyaluronsäure | 0,4 | 0,385 |
| TRIS HCl | 0,480 | |
| TRIS | 0,048 | |
| NaCl | 0,450 | 0,460 |
| pH | 7-7,5 | 7,45 |
| Osmolalität mOsm/Kg | 260-310 | 260 |
| Steril durch filtrieren bei 0,2 µ | Ja | Ja |
| Rest-BDDE ppm | <2 | Übereinstimmend |
| Größe nm | >5 < 200 | Übereinstimmend |

8. Sterile Augentropfen, welche basierend auf 0,2% nanostrukturierter vernetzter Säure, hergestellt nach einem der Ansprüche 1-4 in einem Tris-Tris HCl Puffer, durch filtrieren gewonnen werden mit folgender Zusammensetzung:
| Inhaltsstoffe | Theoretische % Konzentration | Reale % Konzentration |
|---|---|---|
| Nanostrukturierte vernetzte Hyaluronsäure | 0,2 | 0,185 |
| TRIS HCl | 0,480 | 0,480 |
| TRIS | 0,048 | 0,480 |
| NaCl | 0,450 | 0,460 |
| pH | 7-7,5 | 7,45 |
| Osmolalität mOsm/Kg | 260-310 | 265 |
| Steril durch filtrieren bei 0,2 mu | Ja | Ja |
| Rest-BDDE ppm | <2 | Übereinstimmend |
| Größe nm | >5 < 200 | Übereinstimmend |

9. Sterile Augentropfen, welche basierend auf 0,4% w/w nanostrukturierter vernetzter Hyaluronsäure in einem Boratpuffer durch filtrieren bei 0,2 Mikrons gewonnen werden, nach Anspruch 6, zur medizinischen Verwendung.

10. Sterile Augentropfen, welche basierend auf 0,4% w/w nanostrukturierter vernetzter Hyaluronsäure in einem Boratpuffer durch filtrieren bei 0,2 Mikrons gewonnen werden, nach Anspruch 6, zur Verwendung in einem Verfahren zur Behandlung des Syndroms des trockenen Auges.

11. Sterile Augentropfen, welche basierend auf 0,2% w/w nanostrukturierter vernetzter Hyaluronsäure in einem Boratpuffer durch filtrieren bei 0,2 Mikrons gewonnen werden, nach Anspruch 6, zur medizinischen Verwendung.

12. Sterile Augentropfen, welche basierend auf 0,2% w/w nanostrukturierter vernetzter Hyaluronsäure in einem Boratpuffer durch filtrieren bei 0,2 Mikrons gewonnen werden, nach Anspruch 6, zur Verwendung in einem Verfahren zur Behandlung des Syndroms des trockenen Auges.

13. Sterile Augentropfen, welche basierend auf 0,2% w/w nanostrukturierter vernetzter Hyaluronsäure in einem Tris Tris-HCl Puffer durch filtrieren bei 0,2 Mikrons gewonnen werden, nach Anspruch 8, zur medizinischen Verwendung.

14. Sterile Augentropfen, welche basierend auf 0,2% w/w nanostrukturierter vernetzter Hyaluronsäure in einem Tris Tris-HCl Puffer durch filtrieren bei 0,2 Mikrons gewonnen werden, nach Anspruch 8, zur Verwendung in einem Verfahren zur Behandlung des Syndroms des trockenen Auges.

## Revendications

1. Procédé pour préparer de l'acide hyaluronique réticulé qui peut être filtré à 0,2 µ, **caractérisé en ce qu'**il comporte les étapes suivantes :
a) la réalisation d'une réaction de réticulation entre une solution de sel de sodium d'acide hyaluronique dans un milieu basique dans une concentration comprise entre 1 % et 4 % en poids par rapport au poids total de la composition et de BDDE dans une concentration de 20 % par rapport au poids de l'acide hyaluronique, de manière à obtenir un gel dans lequel la concentration finale de sel de sodium d'acide hyaluronique réticulé est comprise entre 4 % et 1 % en poids par rapport au poids total dudit gel ;
b) la neutralisation partielle du gel avec un acide, de préférence de l'HCl, pour l'amener à un pH compris entre 5,5 et 6,5, à une concentration finale d'acide hyaluronique réticulé de 2 % à 0,5 % m/m, qui est égale à la moitié de la concentration initiale d'acide hyaluronique ;
c) le fait de laisser le gel reposer à température ambiante pendant une période d'au moins 6 jours de manière à permettre la réticulation complète du BDDE et la dégradation complète de celui-ci, dans lequel la taille de particule de l'acide hyaluronique réticulé est comprise entre 8 - 50 nm ;
d) la dilution à une concentration comprise entre 0,4 % et 0,1 % m/m dudit gel partiellement neutralisé avec de l'eau distillée.

2. Procédé pour préparer de l'acide hyaluronique réticulé qui peut être filtré à 0,2 µ selon la revendication précédente, **caractérisé en ce que** dans l'étape a) l'alcalinité est comprise entre 1 M et 0,2 M dans le NaOH et la neutralisation partielle subséquente dans l'étape b) a lieu avec l'HCl compris entre 1 M et 0,2 M.

3. Procédé pour préparer un produit ophtalmique avec de l'acide hyaluronique réticulé stérilisé par filtration à 0,2 µ, **caractérisé en ce qu'**il comporte :
la préparation dudit produit dans un tampon de borate par ajout d'acide borique et de tétraborate de sodium décahydraté à l'acide hyaluronique réticulé obtenu selon la revendication 1 ou revendication 2 sous agitation jusqu'à un pH final compris entre 7 et 7,4 ;
l'homogénéisation du produit à 0,4 % m/m avec un homogénéisateur à palettes pendant 10 minutes à une vitesse comprise entre 1 000 et 1 300 tr/min, jusqu'à l'atteinte d'une température finale de 39-40 °C ;
et
la filtration à 0,2 micron du produit homogénéisé pour la stérilisation de celui-ci.

4. Procédé pour préparer un produit ophtalmique qui peut être stérilisé par filtration à 0,2 µ, selon la revendication 3, **caractérisé en ce qu'**il comporte les étapes suivantes :
étape 1 : une solution de 4 % d'hyaluronate de sodium est préparée, à une concentration de NaOH comprise entre 1 et 0,2 M ;
étape 2 : une quantité en poids de BDDE égale à 20 % de sel de sodium d'acide hyaluronique est ajoutée à la solution préparée dans l'étape 1 ;
- mélange pendant 2 minutes ;
- fait de laisser à une température de 30 °C pendant 4-8 heures, de préférence 6 heures, obtenant ainsi une concentration finale de sel de sodium d'acide hyaluronique de 4 % m/m de la solution totale ;
étape 3 : la quantité requise de 1 N de HCl est ajoutée pour amener le gel à un pH compris entre 5,5-6,5, de sorte que la concentration finale du gel d'acide hyaluronique réticulé est de 2 % en poids par rapport au poids total du produit final, éventuellement ajout d'eau ;
étape 4 : le produit ainsi neutralisé sous la forme d'un gel est laissé à température ambiante pendant un temps suffisant pour dégrader complètement le BDDE possiblement présent ;
étape 5 : le gel neutralisé dans l'étape 3 est dilué avec de l'eau distillée pour obtenir la concentration de 0, 40 % ;
- dans lequel le pH est compris entre 5,5-6,5 et l'osmolalité mOsm/Kg est comprise entre 145-155.
étape 6 : au produit dilué à 0,4 %, de l'acide borique, et du tétraborate de sodium décahydraté sont ajoutés de la manière suivante :
- acide borique : dans une quantité suffisante pour obtenir une concentration finale dans le produit comprise entre 0,8 - 0,7 % ;
- tétraborate de sodium : dans une quantité suffisante pour présenter une concentration finale de 0,100 % ;
- laisser sous agitation pendant 5 heures ;
- contrôle du pH : 7-7,4 ;
- osmolalité mOsm/Kg 270-310 ;
étape 7 : le produit à 0,4 % est homogénéisé avec un homogénéisateur à palettes pendant 10 minutes à une vitesse de 1 000-1 300 tr/min, 5 minutes x2, température finale 39-40 °C ;
étape 8 : le produit homogénéisé dans l'étape 7 est filtré avec un filtre de 0,2 micron ; et
le produit est ainsi stérilisé, le pH étant compris entre 7-7,5 et l'osmolalité mOsm/kg : 260-310.

5. Gouttes ophtalmiques stériles obtenues par filtration à 0,2 micron à base d'acide hyaluronique lié nanostructuré préparé selon l'une quelconque des revendications 1-4, présentant la composition suivante :
| Composition | Concentration théorique % | Concentration réelle % |
|---|---|---|
| acide hyaluronique lié nanostructuré | 0,400 | 0,375-0,415 |
| acide borique | 0,775 | 0,712 |
| tétraborate de sodium | 0,120 | 0,110 |
| NaCl | 0,560 | 0,450 - 0,0600 |
| stérile par filtration à 0,2 micron | oui | oui |
| osmolalité mOsm/kg | 270-310 | 310 |
| taille | 5-inférieure à 20 nm | |
| pH | 7,4 | 7-7,5 |
| BDDE ppm | <2 | conforme |

6. Gouttes ophtalmiques stériles obtenues par filtration à base d'acide hyaluronique lié nanostructuré préparé selon l'une quelconque des revendications 1-4 avec 0,2 % de BDDE, dans un tampon de borate, présentant la composition suivante :
| Ingrédients | Concentration théorique % | Concentration réelle % |
|---|---|---|
| acide hyaluronique lié nanostructuré | 0,2 | 0,190 |
| acide borique | 0,775 | 0,742 |
| tétraborate de sodium décahydraté | 0,120 | 0,117 |
| NaCl | 0,450 | 0,420 |
| pH | 7,4 | 7-7,5 |
| osmolalité mOsm/Kg | 260-310 | 275 |
| stérile par filtration à 0,2 µ | OUI | OUI |
| BDDE résiduel ppm | <2 | conforme |
| taille nm | 5-25 nm <200 | conforme |

7. Gouttes ophtalmiques stériles obtenues par filtration à base d'acide lié nanostructuré à 0,4 % préparé selon l'une quelconque des revendications 1-4 dans un tampon de Tris-Tris HCl présentant la composition suivante :
| Ingrédients | Concentration théorique % | Concentration réelle % |
|---|---|---|
| acide hyaluronique lié nanostructuré | 0,4 | 0,385 |
| TRIS HCl | 0,480 | |
| TRIS | 0,048 | |
| NaCl | 0,450 | 0,460 |
| pH | 7-7,5 | 7,45 |
| osmolalité mOsm/Kg | 260-310 | 260 |
| stérile par filtration à 0,2 micron | oui | oui |
| BDDE résiduel ppm | <2 | conforme |
| taille nm | >5 < 200 | conforme |

8. Gouttes ophtalmiques stériles obtenues par filtration à base d'acide lié nanostructuré à 0,2 % préparé selon l'une quelconque des revendications 1-4 dans un tampon de Tris-Tris-HCl présentant la composition suivante :
| Ingrédients | Concentration théorique % | Concentration réelle % |
|---|---|---|
| acide hyaluronique lié nanostructuré | 0,2 | 0,185 |
| TRIS HCl | 0,480 | 0,480 |
| TRIS | 0,048 | 0,480 |
| NaCl | 0,450 | 0,460 |
| pH | 7-7,5 | 7,45 |
| osmolalité mOsm/Kg | 260-310 | 265 |
| stérile par filtration à 0,2 micron | oui | oui |
| BDDE résiduel ppm | <2 | conforme |
| taille nm | >5 < 200 | conforme |

9. Gouttes ophtalmiques stériles obtenues par filtration à 0,2 micron à base de 0,4 % m/m d'acide hyaluronique lié nanostructuré dans un tampon de borate selon la revendication 6, à usage médical.

10. Gouttes ophtalmiques stériles obtenues par filtration à 0,2 micron à base d'acide hyaluronique lié nanostructuré à 0,4 % m/m dans un tampon de borate selon la revendication 6, pour son utilisation dans un procédé pour le traitement du syndrome de l'œil sec.

11. Gouttes ophtalmiques stériles obtenues par filtration à 0,2 micron à base d'acide hyaluronique lié nanostructuré à 0,2 % m/m dans un tampon de borate selon la revendication 6 pour un usage médical.

12. Gouttes ophtalmiques stériles obtenues par filtration à 0,2 micron à base d'acide hyaluronique lié nanostructuré à 0,2 % m/m dans un tampon de borate selon la revendication 6 pour son utilisation dans un procédé pour le traitement du syndrome de l'œil sec.

13. Gouttes ophtalmiques stériles obtenues par filtration à 0,2 micron à base d'acide hyaluronique lié nanostructuré à 0,2 % m/m dans un tampon de Tris Tris-HCl selon la revendication 8 pour un usage médical.

14. Gouttes ophtalmiques stériles obtenues par filtration à 0,2 micron à base d'acide hyaluronique lié nanostructuré à 0,2 % m/m dans un tampon de Tris Tris-HCl selon la revendication 8 pour son utilisation dans un procédé pour le traitement du syndrome de l'œil sec.
